# EUROPEAN PATENT SPECIFICATION

(11) **EP 2 488 546 B1**
(45) Date of publication and mention of the grant of the patent: **31.12.2014**
(21) Application number: 09764333.2
(22) Date of filing: 15.10.2009
(51) Int. Cl.: C07K 14/00, B82B 3/00

(54) **SELF-ASSEMBLED STRUCTURES COMPOSED OF SINGLE POLYPEPTIDE COMPRISING AT LEAST THREE COILED-COIL FORMING ELEMENTS**
SELBSTANORDNENDE STRUKUTREN AUS EINZELPOLYPEPTIDEN MIT MINDESTENS DREI COILED-COIL-BILDENDEN ELEMENTEN
STRUCTURES AUTO-ASSEMBLÉES COMPOSÉES D'UN POLYPEPTIDE UNIQUE COMPRENANT AU MOINS TROIS ÉLÉMENTS FORMANT UNE SUPER-HÉLICE

(43) Date of publication of application: 22.08.2012
(73) Proprietor: Kemijski Institut, 1000 Ljubljana (SI)
(72) Inventor: JERALA, Roman, 1000 Ljubljana (SI); FEKONJA, Ota, 1000 Ljubljana (SI); POHAR, Jelka, 4244 Podnart (SI); GRADISAR, Helena, 1000 Ljubljana (SI); BENCINA, Mojca, 1000 Ljubljana (SI); HAFNER BRATKOVIC, Iva, 1000 Ljubljana (SI); BREMSAK, Robert, 1218 Komenda (SI); MIKLAVIC, Spela, 1260 Ljubljana-Polje (SI); JELERCIC, Urska, 5270 Ajdovscina (SI); LUKAN, Anja, 1234 Menges (SI); DOLES, Tibor, 6230 Postojna (SI); BOZIC, Sabina, 6333 Secovlje (SI); VERCE, Marko, 1215 Medvode (SI); DEBELJAK, Nika, 4220 Skofja Loka (SI); FRIEDERICH, Jozefa, 1000 Ljubljana (SI)
(74) Representative: Grünecker, Kinkeldey, Stockmair & Schwanhäusser
(86) International application number: PCT/SI2009/000053
(87) International publication number: WO 2011/046521

(56) References cited:
- WO-A1-01/21646
- WO-A2-01/87919
- US-A- 5 824 483
- US-A1- 2003 198 956
- US-B2- 7 179 784
- BROMLEY ELIZABETH H C ET AL: "Designed alpha-helical tectons for constructing multicomponent synthetic biological systems." JOURNAL OF THE AMERICAN CHEMICAL SOCIETY 28 JAN 2009, vol. 131, no. 3, 28 January 2009 (2009-01-28), pages 928-930, XP002574343 ISSN: 1520-5126
- ALBERTI S ET AL: "GENETIC ANALYSIS OF THE LEUCINE HEPTAD REPEATS OF LAC REPRESSOR: EVIDENCE FOR A 4-HELICAL BUNDLE" EMBO JOURNAL, OXFORD UNIVERSITY PRESS, SURREY, GB, vol. 12, no. 8, 1 January 1993 (1993-01-01), pages 3227-3236, XP002949119 ISSN: 0261-4189
- HAGEMANN U B ET AL: "Selectional and Mutational Scope of Peptides Sequestering the Jun-Fos Coiled-Coil Domain" JOURNAL OF MOLECULAR BIOLOGY, LONDON, GB, vol. 381, no. 1, 1 August 2008 (2008-08-01), pages 73-88, XP022832431 ISSN: 0022-2836 [retrieved on 2008-07-09] cited in the application
- KYLE STUART ET AL: "Production of self-assembling biomaterials for tissue engineering." TRENDS IN BIOTECHNOLOGY JUL 2009, vol. 27, no. 7, July 2009 (2009-07), pages 423-433, XP002574344 ISSN: 1879-3096
- ULIJN REIN V ET AL: "Designing peptide based nanomaterials." CHEMICAL SOCIETY REVIEWS APR 2008, vol. 37, no. 4, April 2008 (2008-04), pages 664-675, XP002574345 ISSN: 0306-0012
- ATAMAN-ONAL Y ET AL: "Surfactant-free anionic PLA nanoparticles coated with HIV-1 p24 protein induced enhanced cellular and humoral immune responses in various animal models" JOURNAL OF CONTROLLED RELEASE, ELSEVIER, AMSTERDAM, NL, vol. 112, no. 2, 15 May 2006 (2006-05-15), pages 175-185, XP024957498 ISSN: 0168-3659 [retrieved on 2006-05-15]
- DUBLIN STEVEN ET AL: "Engineering responsive mechanisms to control the assembly of peptide-based nanostructures." BIOCHEMICAL SOCIETY TRANSACTIONS AUG 2009, vol. 37, no. Pt 4, August 2009 (2009-08), pages 653-659, XP002574346 ISSN: 1470-8752
- Karen A. Mcallister ET AL: "Using [alpha]-Helical Coiled-Coils to Design Nanostructured Metalloporphyrin Arrays", Journal of the American Chemical Society, vol. 130, no. 36, 10 September 2008 (2008-09-10), pages 11921-11927, XP055067724, ISSN: 0002-7863, DOI: 10.1021/ja800697g
- He Dong ET AL: "Self-Assembly of [alpha]-Helical Coiled Coil Nanofibers", Journal of the American Chemical Society, vol. 130, no. 41, 15 October 2008 (2008-10-15), pages 13691-13695, XP055067726, ISSN: 0002-7863, DOI: 10.1021/ja8037323

### SEQUENCE LISTING

<110> Kemijski Institut
<120> Self-assembled structures composed of single polypeptide comprising at least three coiled-coil forming elements
<130> EP81644FZSZpau
<140> 09 764 333.2
   <141> 2009-10-15
<150> PCT/SI2009/000053
   <151> 2009-10-15
<160> 38
<210> 1
   <211> 93
   <212> DNA
   <213> synthetic sequence
<220>
   <221> CDS
   <222> (1)..(93)
   <223> APH-1
<400> 1
   atg aaa cag ctg gaa aaa gaa ctg caa gca atc gaa aaa cag ctg gca      48
   Met Lys Gln Leu Glu Lys Glu Leu Gln Ala Ile Glu Lys Gln Leu Ala
   1               5                   10                  15
   caa ctg caa tgg aag gct caa gct cgc aag aaa aag ctg gca cag          93
   Gln Leu Gln Trp Lys Ala Gln Ala Arg Lys Lys Lys Leu Ala Gln
               20                  25                  30
<210> 2
   <211> 31
   <212> PRT
   <213> synthetic sequence
<400> 2
   Met Lys Gln Leu Glu Lys Glu Leu Gln Ala Ile Glu Lys Gln Leu Ala
   1               5                   10                  15
   Gln Leu Gln Trp Lys Ala Gln Ala Arg Lys Lys Lys Leu Ala Gln
               20                  25                  30
<210> 3
   <211> 135
   <212> DNA
   <213> synthetic sequence
<220>
   <221> CDS
   <222> (1)..(135)
   <223> APH
<400> 3
   atg aaa cag ctg gaa aaa gag ctg aaa cag tta gaa aaa gaa ctg caa      48
   Met Lys Gln Leu Glu Lys Glu Leu Lys Gln Leu Glu Lys Glu Leu Gln
   1               5                   10                  15
   gca att gaa aaa cag ctg gca cag ctg caa tgg aaa gca cag gca cgt      96
   Ala Ile Glu Lys Gln Leu Ala Gln Leu Gln Trp Lys Ala Gln Ala Arg
               20                  25                  30
   aaa aaa aaa ctg gcc cag ctg aaa aaa aaa ctt cag gcc                 135
   Lys Lys Lys Leu Ala Gln Leu Lys Lys Lys Leu Gln Ala
           35                  40                  45
<210> 4
   <211> 45
   <212> PRT
   <213> synthetic sequence
<400> 4
   Met Lys Gln Leu Glu Lys Glu Leu Lys Gln Leu Glu Lys Glu Leu Gln
   1               5                   10                  15
   Ala Ile Glu Lys Gln Leu Ala Gln Leu Gln Trp Lys Ala Gln Ala Arg
               20                  25                  30
   Lys Lys Lys Leu Ala Gln Leu Lys Lys Lys Leu Gln Ala
           35                  40                  45
<210> 5
   <211> 111
   <212> DNA
   <213> synthetic protein
<220>
   <221> CDS
   <222> (1)..(111)
   <223> BCR
<400> 5
   atg gat att gaa cag gaa ctg gaa cgc gca aaa gca agc att cgt cgt      48
   Met Asp Ile Glu Gln Glu Leu Glu Arg Ala Lys Ala Ser Ile Arg Arg
   1               5                   10                  15
   ctg gaa cag gaa gtt aat caa gaa cgt agc cgt atg gca tat ctg caa      96
   Leu Glu Gln Glu Val Asn Gln Glu Arg Ser Arg Met Ala Tyr Leu Gln
               20                  25                  30
   acc ctg ctg gca aaa                                                  111
   Thr Leu Leu Ala Lys
           35
<210> 6
   <211> 37
   <212> PRT
   <213> synthetic protein
<400> 6
   Met Asp Ile Glu Gln Glu Leu Glu Arg Ala Lys Ala Ser Ile Arg Arg
   1               5                   10                  15
   Leu Glu Gln Glu Val Asn Gln Glu Arg Ser Arg Met Ala Tyr Leu Gln
               20                  25                  30
   Thr Leu Leu Ala Lys
           35
<210> 7
   <211> 99
   <212> DNA
   <213> Synthetic
<220>
   <221> CDS
   <222> (1) .. (99)
   <223> GCN
<400> 7
   cgt atg aaa cag ctg gaa gat aaa atc gag gag ctg ctg tct aag atc      48
   Arg Met Lys Gln Leu Glu Asp Lys Ile Glu Glu Leu Leu Ser Lys Ile
   1               5                   10                  15
   tac cac ctg gaa aac gaa att gct cgc ctg aaa aag ctg att ggt gaa      96
   Tyr His Leu Glu Asn Glu Ile Ala Arg Leu Lys Lys Leu Ile Gly Glu
               20                  25                  30
   cgc                                                                  99
   Arg
<210> 8
   <211> 33
   <212> PRT
   <213> Synthetic
<400> 8
   Arg Met Lys Gln Leu Glu Asp Lys Ile Glu Glu Leu Leu Ser Lys Ile
   1               5                   10                  15
   Tyr His Leu Glu Asn Glu Ile Ala Arg Leu Lys Lys Leu Ile Gly Glu
               20                  25                  30
   Arg
<210> 9
   <211> 102
   <212> DNA
   <213> Synthetic
<220>
   <221> CDS
   <222> (1)..(102)
   <223> H1
<400> 9
   atg tcc cca gaa gat gaa atc caa gca ctg aaa cag gaa aat gcg caa      48
   Met Ser Pro Glu Asp Glu Ile Gln Ala Leu Lys Gln Glu Asn Ala Gln
   1               5                   10                  15
   ctg aaa gag aaa atc caa caa ctg gaa cag gaa att cag gct ctg aag      96
   Leu Lys Glu Lys Ile Gln Gln Leu Glu Gln Glu Ile Gln Ala Leu Lys
               20                  25                  30
   tat ggc                                                             102
   Tyr Gly
<210> 10
   <211> 34
   <212> PRT
   <213> Synthetic
<400> 10
   Met Ser Pro Glu Asp Glu Ile Gln Ala Leu Lys Gln Glu Asn Ala Gln
   1 5 10 15
   Leu Lys Glu Lys Ile Gln Gln Leu Glu Gln Glu Ile Gln Ala Leu Lys
   20 25 30
   Tyr Gly
<210> 11
   <211> 99
   <212> DNA
   <213> Synthetic
<220>
   <221> CDS
   <222> (1) .. (99)
   <223> P1
<400> 11
   agc cca gaa gac gaa att cag gca ctg gaa gaa gaa aat gct caa ctg 48
   Ser Pro Glu Asp Glu Ile Gln Ala Leu Glu Glu Glu Asn Ala Gln Leu
   1 5 10 15
   gaa cag gaa aac gcg gcg ctg gaa gaa gaa atc gca cag ctg gaa tac 96
   Glu Gln Glu Asn Ala Ala Leu Glu Glu Glu Ile Ala Gln Leu Glu Tyr
   20 25 30
   ggc 99
   Gly
<210> 12
   <211> 33
   <212> PRT
   <213> Synthetic
<400> 12
   Ser Pro Glu Asp Glu Ile Gln Ala Leu Glu Glu Glu Asn Ala Gln Leu
   1 5 10 15
   Glu Gln Glu Asn Ala Ala Leu Glu Glu Glu Ile Ala Gln Leu Glu Tyr
   20 25 30
   Gly
<210> 13
   <211> 99
   <212> DNA
   <213> Synthetic
<220>
   <221> CDS
   <222> (1)..(99)
   <223> P2
<400> 13
   tct cca gaa gac aaa atc gca cag ctg aaa gaa aag aac gcg gcc ctg       48
   Ser Pro Glu Asp Lys Ile Ala Gln Leu Lys Glu Lys Asn Ala Ala Leu
   1               5                   10                  15
   aaa gaa aag aat caa cag ctg aag gag aaa atc caa gca ctg aaa tat       96
   Lys Glu Lys Asn Gln Gln Leu Lys Glu Lys Ile Gln Ala Leu Lys Tyr
               20                  25                  30
   ggc                                                                   99
   Gly
<210> 14
   <211> 33
   <212> PRT
   <213> Synthetic
<400> 14
   Ser Pro Glu Asp Lys Ile Ala Gln Leu Lys Glu Lys Asn Ala Ala Leu
   1               5                   10                  15
   Lys Glu Lys Asn Gln Gln Leu Lys Glu Lys Ile Gln Ala Leu Lys Tyr
               20                  25                  30
   Gly
<210> 15
   <211> 99
   <212> DNA
   <213> Synthetic
<220>
   <221> CDS
   <222> (1)..(99)
   <223> P3
<400> 15
   tcc ccg gaa gat gag atc cag caa ctg gaa gaa gaa atc gct cag ctg       48
   Ser Pro Glu Asp Glu Ile Gln Gln Leu Glu Glu Glu Ile Ala Gln Leu
   1               5                   10                  15
   gaa cag aaa aac gca gcg ctg aaa gag aaa aac cag gcg ctg aaa tac       96
   Glu Gln Lys Asn Ala Ala Leu Lys Glu Lys Asn Gln Ala Leu Lys Tyr
               20                  25                  30
   ggt                                                                   99
   Gly
<210> 16
   <211> 33
   <212> PRT
   <213> Synthetic
<400> 16
   Ser Pro Glu Asp Glu Ile Gln Gln Leu Glu Glu Glu Ile Ala Gln Leu
   1 5 10 15
   Glu Gln Lys Asn Ala Ala Leu Lys Glu Lys Asn Gln Ala Leu Lys Tyr
   20 25 30
   Gly
<210> 17
   <211> 99
   <212> DNA
   <213> Synthetic
<220>
   <221> CDS
   <222> (1)..(99)
   <223> P4
<400> 17
   agc ccg gaa gat aaa att gct cag ctg aaa caa aaa atc caa gcg ctg 48
   Ser Pro Glu Asp Lys Ile Ala Gln Leu Lys Gln Lys Ile Gln Ala Leu
   1 5 10 15
   aaa cag gaa aac cag cag ctg gaa gag gaa aac gcc gca ctg gaa tat 96
   Lys Gln Glu Asn Gln Gln Leu Glu Glu Glu Asn Ala Ala Leu Glu Tyr
   20 25 30
   ggt 99
   Gly
<210> 18
   <211> 33
   <212> PRT
   <213> Synthetic
<400> 18
   Ser Pro Glu Asp Lys Ile Ala Gln Leu Lys Gln Lys Ile Gln Ala Leu
   1 5 10 15
   Lys Gln Glu Asn Gln Gln Leu Glu Glu Glu Asn Ala Ala Leu Glu Tyr
   20 25 30
   Gly
<210> 19
   <211> 99
   <212> DNA
   <213> Synthetic
<220>
   <221> CDS
   <222> (1)..(99)
   <223> P5
<400> 19
   tct cct gaa gac gaa aac gca gct ctg gaa gag aaa att gca caa ctg        48
   Ser Pro Glu Asp Glu Asn Ala Ala Leu Glu Glu Lys Ile Ala Gln Leu
   1               5                   10                  15
   aaa caa aag aac gcg gca ctg aaa gaa gaa att caa gca ctg gaa tat        96
   Lys Gln Lys Asn Ala Ala Leu Lys Glu Glu Ile Gln Ala Leu Glu Tyr
               20                  25                  30
   ggc                                                                    99
   Gly
<210> 20
   <211> 33
   <212> PRT
   <213> Synthetic
<400> 20
   Ser Pro Glu Asp Glu Asn Ala Ala Leu Glu Glu Lys Ile Ala Gln Leu
   1               5                   10                  15
   Lys Gln Lys Asn Ala Ala Leu Lys Glu Glu Ile Gln Ala Leu Glu Tyr
               20                  25                  30
   Gly
<210> 21
   <211> 99
   <212> DNA
   <213> Synthetic
<220>
   <221> CDS
   <222> (1)..(99)
   <223> P6
<400> 21
   agc ccg gaa gat aaa aac gcc gct ctg aaa gag gaa atc cag gcg ctg        48
   Ser Pro Glu Asp Lys Asn Ala Ala Leu Lys Glu Glu Ile Gln Ala Leu
   1               5                   10                  15
   gaa gaa gaa aac cag gct ctg gaa gag aaa atc gca cag ctg aaa tat        96
   Glu Glu Glu Asn Gln Ala Leu Glu Glu Lys Ile Ala Gln Leu Lys Tyr
               20                  25                  30
   ggt                                                                    99
   Gly
<210> 22
   <211> 33
   <212> PRT
   <213> Synthetic
<400> 22
   Ser Pro Glu Asp Lys Asn Ala Ala Leu Lys Glu Glu Ile Gln Ala Leu
   1 5 10 15
   Glu Glu Glu Asn Gln Ala Leu Glu Glu Lys Ile Ala Gln Leu Lys Tyr
   20 25 30
   Gly
<210> 23
   <211> 99
   <212> DNA
   <213> Synthetic
<220>
   <221> CDS
   <222> (1)..(99)
   <223> P7
<400> 23
   tcc ccg gag gat gag atc cag gcg ctg gaa gaa aag aac gcc cag ctg 48
   Ser Pro Glu Asp Glu Ile Gln Ala Leu Glu Glu Lys Asn Ala Gln Leu
   1 5 10 15
   aag cag gaa att gcg gca ctg gaa gag aag aac cag gcc ctg aag tac 96
   Lys Gln Glu Ile Ala Ala Leu Glu Glu Lys Asn Gln Ala Leu Lys Tyr
   20 25 30
   ggt 99
   Gly
<210> 24
   <211> 33
   <212> PRT
   <213> Synthetic
<400> 24
   Ser Pro Glu Asp Glu Ile Gln Ala Leu Glu Glu Lys Asn Ala Gln Leu
   1 5 10 15
   Lys Gln Glu Ile Ala Ala Leu Glu Glu Lys Asn Gln Ala Leu Lys Tyr
   20 25 30
   Gly
<210> 25
   <211> 99
   <212> DNA
   <213> Synthetic
<220>
   <221> CDS
   <222> (1) .. (99)
   <223> P8
<400> 25
   tcc ccg gaa gac aaa atc gct cag ctg aaa gaa gaa aac cag cag ctg       48
   Ser Pro Glu Asp Lys Ile Ala Gln Leu Lys Glu Glu Asn Gln Gln Leu
   1               5                   10                  15
   gaa caa aag att cag gcc ctg aag gag gaa aac gca gct ctg gaa tac       96
   Glu Gln Lys Ile Gln Ala Leu Lys Glu Glu Asn Ala Ala Leu Glu Tyr
               20                  25                  30
   ggc                                                                   99
   Gly
<210> 26
   <211> 33
   <212> PRT
   <213> Synthetic
<400> 26
   Ser Pro Glu Asp Lys Ile Ala Gln Leu Lys Glu Glu Asn Gln Gln Leu
   1               5                   10                  15
   Glu Gln Lys Ile Gln Ala Leu Lys Glu Glu Asn Ala Ala Leu Glu Tyr
               20                  25                  30
   Gly
<210> 27
   <211> 312
   <212> DNA
   <213> Synthetic
<220>
   <221> CDS
   <222> (1)..(312)
   <223> K2-P1-GCN-P2
<400> 27
   atg agc cca gaa gac gaa att cag gca ctg gaa gaa gaa aat gct caa      48
   Met Ser Pro Glu Asp Glu Ile Gln Ala Leu Glu Glu Glu Asn Ala Gln
   1               5                   10                  15
   ctg gaa cag gaa aac gcg gcg ctg gaa gaa gaa atc gca cag ctg gaa      96
   Leu Glu Gln Glu Asn Ala Ala Leu Glu Glu Glu Ile Ala Gln Leu Glu
               20                  25                  30
   tac ggc tcc ggc cgt atg aaa cag ctg gaa gat aaa atc gag gag ctg      144
   Tyr Gly Ser Gly Arg Met Lys Gln Leu Glu Asp Lys Ile Glu Glu Leu
           35                  40                  45
   ctg tct aag atc tac cac ctg gaa aac gaa att gct cgc ctg aaa aag      192
   Leu Ser Lys Ile Tyr His Leu Glu Asn Glu Ile Ala Arg Leu Lys Lys
       50                  55                  60
   ctg att ggt gaa cgc tcc ggc tct cca gaa gac aaa atc gca cag ctg      240
   Leu Ile Gly Glu Arg Ser Gly Ser Pro Glu Asp Lys Ile Ala Gln Leu
   65                  70                  75                  80
   aaa gaa aag aac gcg gcc ctg aaa gaa aag aat caa cag ctg aag gag      288
   Lys Glu Lys Asn Ala Ala Leu Lys Glu Lys Asn Gln Gln Leu Lys Glu
                   85                  90                  95
   aaa atc caa gca ctg aaa tat ggc                                      312
   Lys Ile Gln Ala Leu Lys Tyr Gly
               100
<210> 28
   <211> 104
   <212> PRT
   <213> Synthetic
<400> 28
   Met Ser Pro Glu Asp Glu Ile Gln Ala Leu Glu Glu Glu Asn Ala Gln
   1               5                   10                  15
   Leu Glu Gln Glu Asn Ala Ala Leu Glu Glu Glu Ile Ala Gln Leu Glu
               20                  25                  30
   Tyr Gly Ser Gly Arg Met Lys Gln Leu Glu Asp Lys Ile Glu Glu Leu
           35                  40                  45
   Leu Ser Lys Ile Tyr His Leu Glu Asn Glu Ile Ala Arg Leu Lys Lys
       50                  55                  60
   Leu Ile Gly Glu Arg Ser Gly Ser Pro Glu Asp Lys Ile Ala Gln Leu
   65                  70                  75                  80
   Lys Glu Lys Asn Ala Ala Leu Lys Glu Lys Asn Gln Gln Leu Lys Glu
                   85                  90                  95
   Lys Ile Gln Ala Leu Lys Tyr Gly
               100
<210> 29
   <211> 312
   <212> DNA
   <213> Synthetic
<220>
   <221> CDS
   <222> (1)..(312)
   <223> K22 - P2-GCN-P1
<400> 29
   atg tct cca gaa gac aaa atc gca cag ctg aaa gaa aag aac gcg gcc      48
   Met Ser Pro Glu Asp Lys Ile Ala Gln Leu Lys Glu Lys Asn Ala Ala
   1               5                   10                  15
   ctg aaa gaa aag aat caa cag ctg aag gag aaa atc caa gca ctg aaa      96
   Leu Lys Glu Lys Asn Gln Gln Leu Lys Glu Lys Ile Gln Ala Leu Lys
               20                  25                  30
   tat ggc tcc ggc cgt atg aaa cag ctg gaa gat aaa atc gag gag ctg      144
   Tyr Gly Ser Gly Arg Met Lys Gln Leu Glu Asp Lys Ile Glu Glu Leu
           35                  40                  45
   ctg tct aag atc tac cac ctg gaa aac gaa att gct cgc ctg aaa aag      192
   Leu Ser Lys Ile Tyr His Leu Glu Asn Glu Ile Ala Arg Leu Lys Lys
       50                  55                  60
   ctg att ggt gaa cgc tcc ggc agc cca gaa gac gaa att cag gca ctg      240
   Leu Ile Gly Glu Arg Ser Gly Ser Pro Glu Asp Glu Ile Gln Ala Leu
   65                  70                  75                  80
   gaa gaa gaa aat gct caa ctg gaa cag gaa aac gcg gcg ctg gaa gaa      288
   Glu Glu Glu Asn Ala Gln Leu Glu Gln Glu Asn Ala Ala Leu Glu Glu
                   85                  90                  95
   gaa atc gca cag ctg gaa tac ggc                                      312
   Glu Ile Ala Gln Leu Glu Tyr Gly
               100
<210> 30
   <211> 104
   <212> PRT
   <213> Synthetic
<400> 30
   Met Ser Pro Glu Asp Lys Ile Ala Gln Leu Lys Glu Lys Asn Ala Ala
   1               5                   10                  15
   Leu Lys Glu Lys Asn Gln Gln Leu Lys Glu Lys Ile Gln Ala Leu Lys
               20                  25                  30
   Tyr Gly Ser Gly Arg Met Lys Gln Leu Glu Asp Lys Ile Glu Glu Leu
           35                  40                  45
   Leu Ser Lys Ile Tyr His Leu Glu Asn Glu Ile Ala Arg Leu Lys Lys
       50                  55                  60
   Leu Ile Gly Glu Arg Ser Gly Ser Pro Glu Asp Glu Ile Gln Ala Leu
   65                  70                  75                  80
   Glu Glu Glu Asn Ala Gln Leu Glu Gln Glu Asn Ala Ala Leu Glu Glu
                   85                  90                  95
   Glu Ile Ala Gln Leu Glu Tyr Gly
           100
<210> 31
   <211> 312
   <212> DNA
   <213> Synthetic
<220>
   <221> CDS
   <222> (1) .. (312)
   <223> K3 - P1-P2-GCN
<400> 31
   atg agc cca gaa gac gaa att cag gca ctg gaa gaa gaa aat gct caa      48
   Met Ser Pro Glu Asp Glu Ile Gln Ala Leu Glu Glu Glu Asn Ala Gln
   1               5                   10                  15
   ctg gaa cag gaa aac gcg gcg ctg gaa gaa gaa atc gca cag ctg gaa      96
   Leu Glu Gln Glu Asn Ala Ala Leu Glu Glu Glu Ile Ala Gln Leu Glu
               20                  25                  30
   tac ggc tcc ggc tct cca gaa gac aaa atc gca cag ctg aaa gaa aag      144
   Tyr Gly Ser Gly Ser Pro Glu Asp Lys Ile Ala Gln Leu Lys Glu Lys
           35                  40                  45
   aac gcg gcc ctg aaa gaa aag aat caa cag ctg aag gag aaa atc caa      192
   Asn Ala Ala Leu Lys Glu Lys Asn Gln Gln Leu Lys Glu Lys Ile Gln
       50                  55                  60
   gca ctg aaa tat ggc tcc ggc cgt atg aaa cag ctg gaa gat aaa atc      240
   Ala Leu Lys Tyr Gly Ser Gly Arg Met Lys Gln Leu Glu Asp Lys Ile
   65                  70                  75                  80
   gag gag ctg ctg tct aag atc tac cac ctg gaa aac gaa att gct cgc      288
   Glu Glu Leu Leu Ser Lys Ile Tyr His Leu Glu Asn Glu Ile Ala Arg
                   85                  90                  95
   ctg aaa aag ctg att ggt gaa cgc                                      312
   Leu Lys Lys Leu Ile Gly Glu Arg
               100
<210> 32
   <211> 104
   <212> PRT
   <213> Synthetic
<400> 32
   Met Ser Pro Glu Asp Glu Ile Gln Ala Leu Glu Glu Glu Asn Ala Gln
   1               5                   10                  15
   Leu Glu Gln Glu Asn Ala Ala Leu Glu Glu Glu Ile Ala Gln Leu Glu
               20                  25                  30
   Tyr Gly Ser Gly Ser Pro Glu Asp Lys Ile Ala Gln Leu Lys Glu Lys
           35                  40                  45
   Asn Ala Ala Leu Lys Glu Lys Asn Gln Gln Leu Lys Glu Lys Ile Gln
       50                  55                  60
   Ala Leu Lys Tyr Gly Ser Gly Arg Met Lys Gln Leu Glu Asp Lys Ile
   65                  70                  75                  80
   Glu Glu Leu Leu Ser Lys Ile Tyr His Leu Glu Asn Glu Ile Ala Arg
                   85                  90                  95
   Leu Lys Lys Leu Ile Gly Glu Arg
               100
<210> 33
   <211> 306
   <212> DNA
   <213> Synthetic
<220>
   <221> CDS
   <222> (1)..(306)
   <223> P1-APH1-P2
<400> 33
   atg agc cca gaa gac gaa att cag gca ctg gaa gaa gaa aat gct caa        48
   Met Ser Pro Glu Asp Glu Ile Gln Ala Leu Glu Glu Glu Asn Ala Gln
   1               5                   10                  15
   ctg gaa cag gaa aac gcg gcg ctg gaa gaa gaa atc gca cag ctg gaa        96
   Leu Glu Gln Glu Asn Ala Ala Leu Glu Glu Glu Ile Ala Gln Leu Glu
               20                  25                  30
   tac ggc tcc ggc atg aaa cag ctg gaa aaa gaa ctg caa gca atc gaa       144
   Tyr Gly Ser Gly Met Lys Gln Leu Glu Lys Glu Leu Gln Ala Ile Glu
           35                  40                  45
   aaa cag ctg gca caa ctg caa tgg aag gct caa gct cgc aag aaa aag       192
   Lys Gln Leu Ala Gln Leu Gln Trp Lys Ala Gln Ala Arg Lys Lys Lys
       50                  55                  60
   ctg gca cag tcc ggc tct cca gaa gac aaa atc gca cag ctg aaa gaa       240
   Leu Ala Gln Ser Gly Ser Pro Glu Asp Lys Ile Ala Gln Leu Lys Glu
   65                  70                  75                  80
   aag aac gcg gcc ctg aaa gaa aag aat caa cag ctg aag gag aaa atc       288
   Lys Asn Ala Ala Leu Lys Glu Lys Asn Gln Gln Leu Lys Glu Lys Ile
                   85                  90                  95
   caa gca ctg aaa tat ggc                                               306
   Gln Ala Leu Lys Tyr Gly
               100
<210> 34
   <211> 102
   <212> PRT
   <213> Synthetic
<400> 34
   Met Ser Pro Glu Asp Glu Ile Gln Ala Leu Glu Glu Glu Asn Ala Gln
   1               5                   10                  15
   Leu Glu Gln Glu Asn Ala Ala Leu Glu Glu Glu Ile Ala Gln Leu Glu
               20                  25                  30
   Tyr Gly Ser Gly Met Lys Gln Leu Glu Lys Glu Leu Gln Ala Ile Glu
           35                  40                  45
   Lys Gln Leu Ala Gln Leu Gln Trp Lys Ala Gln Ala Arg Lys Lys Lys
       50                  55                  60
   Leu Ala Gln Ser Gly Ser Pro Glu Asp Lys Ile Ala Gln Leu Lys Glu
   65                  70                  75                  80
   Lys Asn Ala Ala Leu Lys Glu Lys Asn Gln Gln Leu Lys Glu Lys Ile
                   85                  90                  95
   Gln Ala Leu Lys Tyr Gly
               100
<210> 35
   <211> 345
   <212> DNA
   <213> Synthetic
<220>
   <221> CDS
   <222> (1)..(345)
   <223> P1-APH-P2
<400> 35
   agc cca gaa gac gaa att cag gca ctg gaa gaa gaa aat gct caa ctg      48
   Ser Pro Glu Asp Glu Ile Gln Ala Leu Glu Glu Glu Asn Ala Gln Leu
   1               5                   10                  15
   gaa cag gaa aac gcg gcg ctg gaa gaa gaa atc gca cag ctg gaa tac      96
   Glu Gln Glu Asn Ala Ala Leu Glu Glu Glu Ile Ala Gln Leu Glu Tyr
               20                  25                  30
   ggc tcc ggc atg aaa cag ctg gaa aaa gag ctg aaa cag tta gaa aaa     144
   Gly Ser Gly Met Lys Gln Leu Glu Lys Glu Leu Lys Gln Leu Glu Lys
           35                  40                  45
   gaa ctg caa gca att gaa aaa cag ctg gca cag ctg caa tgg aaa gca     192
   Glu Leu Gln Ala Ile Glu Lys Gln Leu Ala Gln Leu Gln Trp Lys Ala
       50                  55                  60
   cag gca cgt aaa aaa aaa ctg gcc cag ctg aaa aaa aaa ctt cag gcc     240
   Gln Ala Arg Lys Lys Lys Leu Ala Gln Leu Lys Lys Lys Leu Gln Ala
   65                  70                  75                  80
   tcc ggc tct cca gaa gac aaa atc gca cag ctg aaa gaa aag aac gcg     288
   Ser Gly Ser Pro Glu Asp Lys Ile Ala Gln Leu Lys Glu Lys Asn Ala
                   85                  90                  95
   gcc ctg aaa gaa aag aat caa cag ctg aag gag aaa atc caa gca ctg     336
   Ala Leu Lys Glu Lys Asn Gln Gln Leu Lys Glu Lys Ile Gln Ala Leu
               100                 105                 110
   aaa tat ggc                                                         345
   Lys Tyr Gly
           115
<210> 36
   <211> 115
   <212> PRT
   <213> Synthetic
<400> 36
   Ser Pro Glu Asp Glu Ile Gln Ala Leu Glu Glu Glu Asn Ala Gln Leu
   1               5                   10                  15
   Glu Gln Glu Asn Ala Ala Leu Glu Glu Glu Ile Ala Gln Leu Glu Tyr
               20                  25                  30
   Gly Ser Gly Met Lys Gln Leu Glu Lys Glu Leu Lys Gln Leu Glu Lys
           35                  40                  45
   Glu Leu Gln Ala Ile Glu Lys Gln Leu Ala Gln Leu Gln Trp Lys Ala
       50                  55                  60
   Gln Ala Arg Lys Lys Lys Leu Ala Gln Leu Lys Lys Lys Leu Gln Ala
   65                  70                  75                  80
   Ser Gly Ser Pro Glu Asp Lys Ile Ala Gln Leu Lys Glu Lys Asn Ala
                   85                  90                  95
   Ala Leu Lys Glu Lys Asn Gln Gln Leu Lys Glu Lys Ile Gln Ala Leu
               100                 105                 110
   Lys Tyr Gly
           115
<210> 37
   <211> 321
   <212> DNA
   <213> Synthetic
<220>
   <221> CDS
   <222> (1) .. (321)
   <223> P1-BCR-P2
<400> 37
   atg agc cca gaa gac gaa att cag gca ctg gaa gaa gaa aat gct caa        48
   Met Ser Pro Glu Asp Glu Ile Gln Ala Leu Glu Glu Glu Asn Ala Gln
   1               5                   10                  15
   ctg gaa cag gaa aac gcg gcg ctg gaa gaa gaa atc gca cag ctg gaa        96
   Leu Glu Gln Glu Asn Ala Ala Leu Glu Glu Glu Ile Ala Gln Leu Glu
               20                  25                  30
   tac ggc tcc ggc gat att gaa cag gaa ctg gaa cgc gca aaa gca agc       144
   Tyr Gly Ser Gly Asp Ile Glu Gln Glu Leu Glu Arg Ala Lys Ala Ser
           35                  40                  45
   att cgt cgt ctg gaa cag gaa gtt aat caa gaa cgt agc cgt atg gca       192
   Ile Arg Arg Leu Glu Gln Glu Val Asn Gln Glu Arg Ser Arg Met Ala
       50                  55                  60
   tat ctg caa acc ctg ctg gca aaa tcc ggc tct cca gaa gac aaa atc       240
   Tyr Leu Gln Thr Leu Leu Ala Lys Ser Gly Ser Pro Glu Asp Lys Ile
   65                  70                  75                  80
   gca cag ctg aaa gaa aag aac gcg gcc ctg aaa gaa aag aat caa cag       288
   Ala Gln Leu Lys Glu Lys Asn Ala Ala Leu Lys Glu Lys Asn Gln Gln
                   85                  90                  95
   ctg aag gag aaa atc caa gca ctg aaa tat ggc                           321
   Leu Lys Glu Lys Ile Gln Ala Leu Lys Tyr Gly
               100                 105
<210> 38
   <211> 107
   <212> PRT
   <213> Synthetic
<400> 38
   Met Ser Pro Glu Asp Glu Ile Gln Ala Leu Glu Glu Glu Asn Ala Gln
   1               5                   10                  15
   Leu Glu Gln Glu Asn Ala Ala Leu Glu Glu Glu Ile Ala Gln Leu Glu
               20                  25                  30
   Tyr Gly Ser Gly Asp Ile Glu Gln Glu Leu Glu Arg Ala Lys Ala Ser
           35                  40                  45
   Ile Arg Arg Leu Glu Gln Glu Val Asn Gln Glu Arg Ser Arg Met Ala
       50                  55                  60
   Tyr Leu Gln Thr Leu Leu Ala Lys Ser Gly Ser Pro Glu Asp Lys Ile
   65                  70                  75                  80
   Ala Gln Leu Lys Glu Lys Asn Ala Ala Leu Lys Glu Lys Asn Gln Gln
                   85                  90                  95
   Leu Lys Glu Lys Ile Gln Ala Leu Lys Tyr Gly
               100                 105

## Description

### Field of invention

The field of this invention is a nanostructure, a new type of structure and material, assembled from a single type of polypeptide chain that comprises at three, four or six separate coiled-coil forming segments. Field of the invention is a polypeptide that forms nanostructures and a DNA coding for the polypeptide forming nanostructures.

### Background of the invention

In recent years DNA has been used to build nanostructures defined by the nucleotide base complementarity. In those structures segments composed of intertwined segments with complementary stretches represent the basic elements that build up the structure. Structures built from DNA include geometric objects, such as tetrahedron, cube, octahedron, dodecahedron or buckyball as well as two dimensional tetragonal or hexagonal lattice or other designed structures (He, Ye, Su, Zhang, Ribbe, Jiang and Mao, Nature 452, 198-202, 2008).

It is relatively easy to prepare and design organization of DNA into self-assembled structure based on base complementarity of single-stranded DNA chains. However for most technological applications polypeptides provide numerous potential advantages in comparison to DNA. Polypeptides are more stable than nucleic acids and above all amino-acid side chains as polypeptide building blocks have many different chemical properties that can provide additional technological properties that include increased stability, define a versatility of tertiary structures, create catalytic sites, create specific interaction/recognition sites, introduce biophysical properties such as optical, electrical, mechanical properties.

Design of polypeptide assembly represents a significant challenge as we can reliably predict or design the tertiary structure only for proteins with homologues of known tertiary structure. This problem can be circumvented by building polypeptide assemblies from building blocks for which we can reliably predict the structure and as well as interactions within polypeptide assemblies.

The idea of protein lattice has been proposed for the two-dimensional arrangement of fusion proteins composed of folded domains that define the symmetry of the lattice (patent application US 2006/0173166 A1). Protein domains that can oligomerize have been combined through a rigid linker between them (patent US 6,756,039 B1). In both of the above cited examples the basic building blocks are folded protein domains that are able to fold independently into defined structures.

Coiled-coil forming segments on the other hand are unable to fold into the final defined structure by themselves, without of interactions with their partner coiled-coil forming segments. Coiled-coil segments, predominantly dimers are components of many proteins as well as of smaller polypeptides. Coiled-coil dimers can assemble in either parallel or antiparallel orientation and can form either heterodimers or homodimers. Coiled-coil forming segments can be structurally dissected into heptads, which typically form two turns of a helix. Characteristics of coiled-coil forming segments is that residues at positions "a" and "d" of the heptad are predominantly hydrophobic residues, typically Ile or Leu and residues at positions "e" and "g" participate in electrostatic interactions, that can prevent formation of undesired combinations. Both of those two types of interactions provide selectivity and inventors realized that they could be used for the formation of desired self-assembled structures. In addition, residues at positions "b", "c" and "f" participate only to the extent of their contribution to the helical propensity and can be used to engineer into the self-assembled structure additional desired properties.

Coiled-coil forming polypeptides have been used to build fibrils, where the polypeptide chain typically comprises two coiled-coil forming segments that are able to twist around each other (patent US 7,045,537 B1).

Extension to three, four or six coiled-coil forming elements in the defined combinations of orientation and type of complementarity as presented by the inventors allow assembly of more complex structures extended into the two or three dimensional nano-cages, nano-networks or nano-tubes.

### Short description of the invention

The invention refers to the following items:
1. Polypeptide comprising any of the following arrangements of coiled-coil forming segments:
   a. parallel heterodimer-forming segment A - parallel homodimer-forming segment - parallel heterodimer-forming segment B,
   b. parallel heterodimer-forming segment A - antiparallel homodimer-forming segment - parallel heterodimer-forming segment B and its cyclic permutations,
   c. antiparallel heterodimer-forming segment A - parallel homodimer-forming segment - antiparallel heterodimer-forming segment B,
   d. antiparallel heterodimer-forming segment A - antiparallel homodimer-forming segment - antiparallel heterodimer-forming segment B,
   e. parallel homodimer-forming segment 1 - antiparallel homodimer-forming segment - parallel homodimer-forming segment 2,
   f. antiparallel homodimer-forming segment 1- parallel homodimer-forming segment - antiparallel homodimer-forming segment 2 and its cyclic permutations,
   g. antiparallel homodimer-forming segment 1 - antiparallel homodimer-forming segment 2 - antiparallel homodimer-forming segment 3,
   h. parallel homodimer-forming segment 1 - parallel homodimer-forming segment 2 - parallel homodimer-forming segment 3 - parallel heterodimer-forming segment A - parallel heterodimer-forming segment B - antiparallel homodimer-forming segment
   i. antiparallel heterodimer-forming segment 1 A - antiparallel heterodimer-forming segment 2A - antiparallel heterodimer-forming segment 1B - antiparallel heterodimer-forming segment 2B, or
   j. antiparallel heterodimer-forming segment 1A - antiparallel heterodimer-forming segment 2A - antiparallel heterodimer-forming segment 3A - antiparallel heterodimer-forming segment 1B - antiparallel heterodimer-forming segment 2B - antiparallel heterodimer-forming segment 3B,
   wherein a pair of heterodimer-forming segment A and heterodimer-forming segment B form a heterodimeric coiled-coil; homodimer-forming segments form coiled-coils with an identical homodimer-forming segment and homodimer-forming segment 1, homodimer-forming segment 2 and homodimer-forming segment 3 form dimers only with themselves (e.g. homodimer-forming segment 1 pairs with another homodimer-forming segment 1), but not with other types of homodimer (e.g. homodimer-forming segment 1 does not pair with homodimer-forming segment 2); and wherein heterodimer-forming segment 1 A and heterodimer-forming segment 1B form antiparallel coiled-coils; and heterodimer-forming segment 2A and heterodimer-forming segment 2B form coiled coils; and heterodimer-forming segment 3A and heterodimer-forming segment 3B form coiled coils;
   further wherein each coiled-coil forming segment comprises two to fifty heptads; and further wherein a non-helical peptide linker is added between the coiled-coil forming segments, the length of said linker being between 1 and 40 amino acid residues.
2. Polypeptide according to item 1, wherein the coiled-coil forming segment is selected from the coiled-coil forming segment of natural protein or designed protein, and is preferentially selected from SEQ ID No.: 2, 4, 6, 8, 10, 12, 14, 16, 18, 20, 22, 24, and 26.
3. Polypeptide according to item 1 or 2, wherein the length of the linker is between 1 and 8 amino-acid residues.
4. Polypeptide according to any of items 1 to 3, prepared by a recombinant method.
5. Nanostructure, comprising the polypeptide of any of items 1-4.
6. Nanostructure according to item 5, wherein the structure is nano-cage, nano-network or nano-tube.
7. Polynucleotide coding for a coiled-coil forming segment, wherein the polynucleotide has a nucleotide sequence selected from SEQ ID Nos. 1, 3, 5, 7, 9, 11, 13, 15, 17, 19, 21, 23, and 25.
8. DNA coding for a polypeptide according to any of items 1 to 4, wherein the DNA is operatively linked to the regulatory elements, promoter and terminator, which drive expression of fusion proteins in the host cells.
9. Procedure for the assembly of a polypeptide nanostructure according to item 5 or 6, comprising the step of dialysing the polypeptide against a dialysis solution for at least 12 hours using slowly decreasing concentrations of a denaturing agent, or against a dialysis solution in which the concentration of the denaturing agent is stepwise decreased in more than five, typically between five and ten, steps.
10. Use of the polypeptide of any of items 1 to 3 for forming the nanostructure of item 5 or 6.
11. Use of the polypeptide of any of items 1 to 3 or the nanostructure of item 5 or 6 as a molecular carrier to encase nanoparticles.
12. The nanostructure according to item 6, wherein the nanostructure is a nano-cage, for use in drug delivery or in a chemical catalysis within the nanocage.
13. The nanostructure according to item 6, wherein the nanostructure is a nano-network, for use in binding specific compounds or as a scaffold.
14. The nanostructure for the use according to item 13, wherein the nano-network is for binding carbon nanotubes, biomineralization, metal binding, formation of conducting circuits, formation of optical assemblies at the nanoscale, combinations with other molecular assemblies, binding of antibodies, binding of nucleic acids, binding of hydrophobic groups for anchoring to the membrane, or ultrafiltration.

### Figure legends:

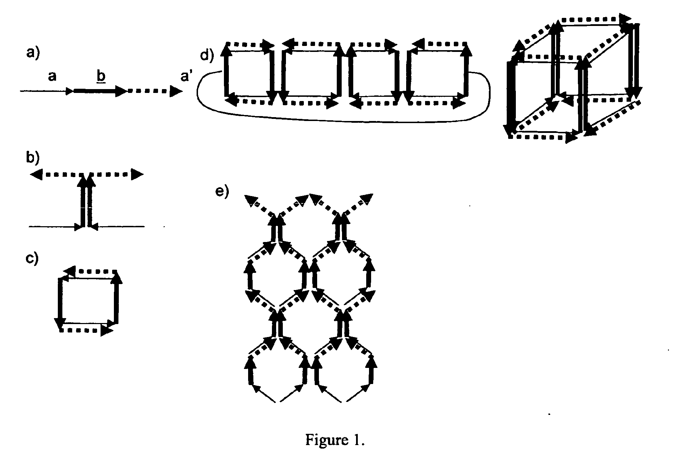

**Figure 1****:** Scheme of the assembly of polypeptide chain comprising three coiled coil segments where two of them form parallel heterodimers (a-a') and one forms a parallel homodimer (b) can self-assemble into the parallelepiped (cuboid) (d) or into a hexagonal network (e). Arrows denote the orientation of the interacting coiled-coil forming segments in the assembly. a and a' denote complementary pair of a parallel heterodimer and b denotes the parallel homodimer.

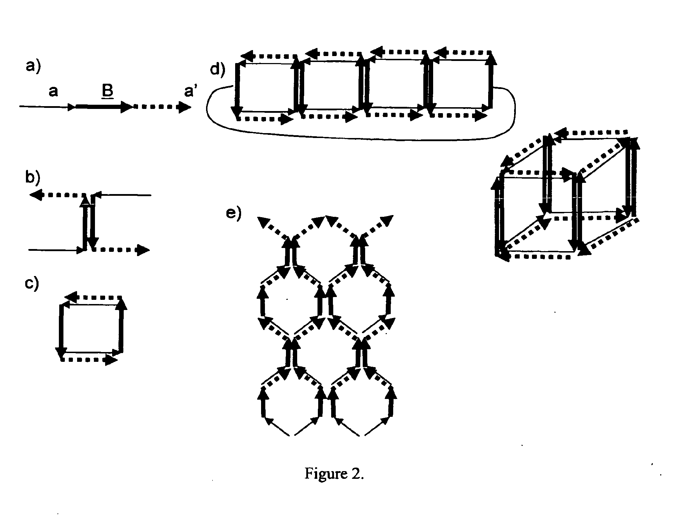

**Figure 2****:** Scheme of the assembly of polypeptide chains comprising three to six coiled-coil forming segments. Polypeptide chain comprising three coiled coil segments where two of them form parallel heterodimers (a-a') and one forms an anti parallel homodimer (b) it can self-assemble into the parallelepiped (cuboid) (d) or into a hexagonal network (e). Arrows denote the orientation of the interacting coiled-coil forming segments in the assembly. a and a' denote complementary pair of a parallel heterodimer and B denotes the antiparallel homodimer

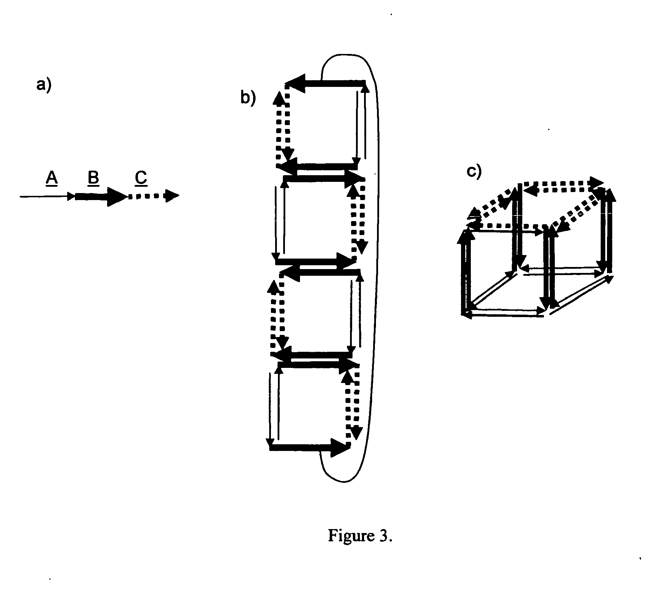

**Figure 3****:** Polypeptide chain comprising three antiparallel homodimers (A, B, C) can self assemble into the parallelepiped (cuboid).

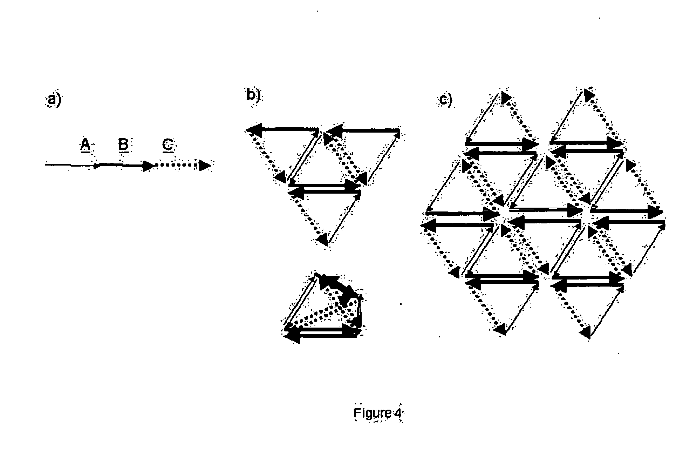

**Figure 4****:** Polypeptide chain comprising three antiparallel homodimers (A, B, C) can self-assemble into tetrahedron and into a trigonal nano-network.

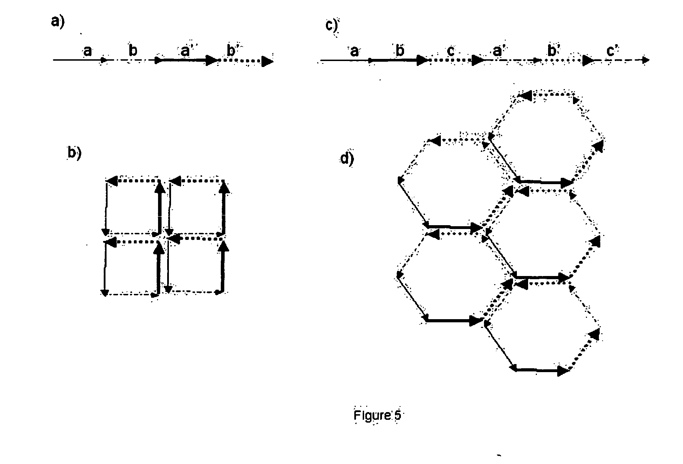

**Figure 5****:** Polypeptide chain comprising four (a) or six (c) antiparallel heterodimers where the coiled-coil segments are formed between a-a', b-b', c-c' and d-d' coiled coil pairing segments can self-assemble into the (b) tetragonal nano-network or (d) hexagonal nano-network.

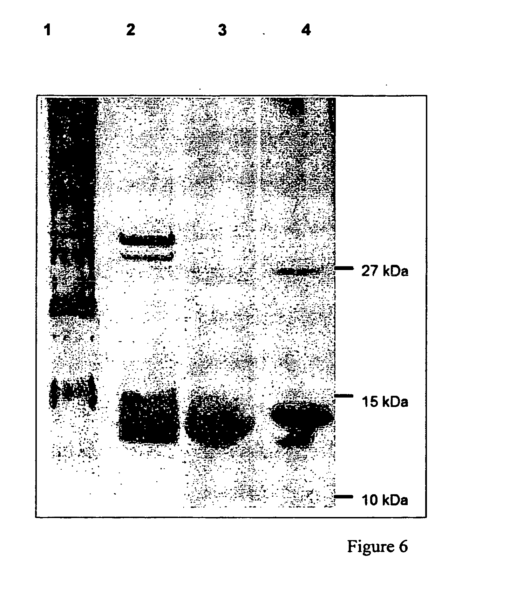

**Figure 6****:** Expression and isolation of polypeptide with SEQ ID: 27 construct. After cell lysis supernatant (lane 1) and insoluble fraction (inclusion bodies, lane 2) were checked for the presence of polypeptide SEQ ID: 28. The protein was mainly present in form of inclusion bodies (lane 2), which were further purified (lane 3). Purified protein (lane 4) was prepared by solubilizing inclusion bodies in 6 M Gdn HCl and purification on nickel-NTA column under denaturing conditions. Additional band at about 27 kDa is a SEQ ID: 28 dimer, which was confirmed by Western blot analysis using anti-His-tag antibodies as primary antibodies (not shown).

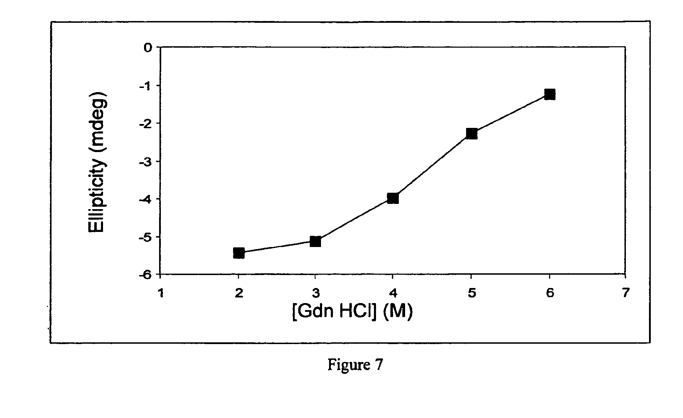

**Figure 7****:** Conditions for refolding of polypeptide with SEQ ID: 28 were found by dissolving polypeptide with SEQ ID: 28 precipitate in 6 M Gdn HCl and dilution to lower concentrations of denaturing agent. Far-UV CD spectra were followed and ellipticity at 222 nm is plotted against concentration of Gdn HCl.

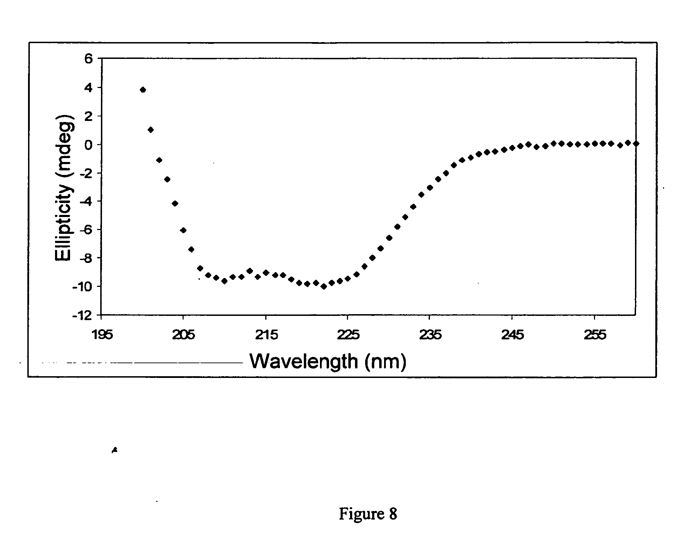

**Figure 8****:** Secondary structure of the assembled polypeptide SEQ ID: No 28 determined from circular dichroism spectra. Isolated protein precipitate was suspended in water at 0.1 mg/ml and circular dichroism spectra were measured in the far UV range, establishing that the coiled-coil is formed.

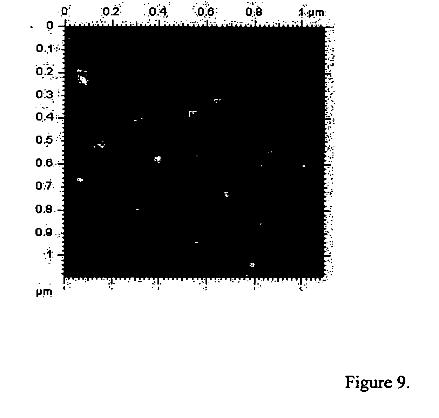

**Figure 9****:** AFM scan of the assembly of nanostructure from polypeptide SEQ ID: no 28 (selfassembled over 12 hours from 5 to 1 M Gdn HCl at protein concentration of 0.5 µg/mL) deposited on mica recorded by Agilent Technologies 5500 Scanning Probe Microscope operating in Acoustic alternative current AFM mode using silicon cantilever PPP-NCH with force constant 42 N/m and tip radius less than 7nm (Nanosensors).

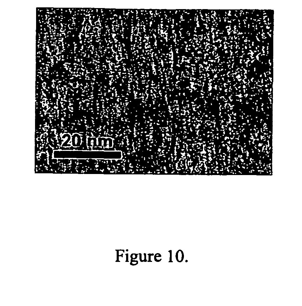

**Figure 10****:** Transmission electron microscope image of the assembled nanonetwork composed of the polypeptide SEQ ID: no 28 (selfassembled over 12 hours from 5 to 1 M Gdn HCl at protein concentration of 0.5 µg/mL). Sample was stained with uranylacetate and visualized on TEM.

### Detailed description

Unless defined otherwise, all technical and scientific terms used herein possess the same meaning as it is commonly known to experts in the field of invention. The terminology to be used in the description of the invention has the purpose of description of a particular segment of the invention and has no intention of limiting the invention. All publications mentioned in the description of the invention are listed as references. In the description of the invention and in the claims, the description is in the singular form, but also includes the plural form, what is not specifically highlighted for ease of understanding.

The basis of invention is a discovery by inventors that defined combinations of three or four or six coiled coil elements within a single polypeptide chain can self-assemble into polypeptide nano-cages or a nano-network with structural elements defined on the nanometer scale. Combination of two coiled-coil forming segments can only lead to the formation of linear, fibrillar assemblies (patent US 7,045,537 B1).

### Design of combinations of coiled-coil forming segments,

Inventors discovered that only some of the possible combinations of three coiled-coil forming segments within a single polypeptide chain can lead to assemblies with defined tertiary structure composed of coiled-coil segments as building blocks. Inventors discovered that for the preparation of nanostructures from a single polypeptide chain each coiled-coil forming segment within this polypeptide chain also has to contain its complement, meaning that there has to be either a homodimeric segment or two coiled-coil sequences of a heterodimer.

Inventors discovered that for three linked coiled-coil forming segments the following combinations of coiled-coil forming segments can be used to assemble nanostructures:
1. parallel heterodimerA - parallel homodimer - parallel heterodimerB
2. parallel heterodimerA - antiparallel homodimer - parallel heterodimerB and its cyclic permutations,
3. antiparallel heterodimerA - parallel homodimer - antiparallel heterodimerB
4. antiparallel heterodimerA - antiparallel homodimer - antiparallel heterodimerB
5. parallel homodimer1 - antiparallel homodimer - parallel homodimer2,
6. antiparallel homodimer1 - parallel homodimer - antiparallel homodimer2 and its cyclic permutations
7. antiparallel homodimer1 - antiparallel homodimer2 - antiparallel homodimer3,
8. parallel homodimer1 - parallel homodimer2 - parallel homodimer3
where heterodimerA and heterodimerB form a paired coiled-coil, while homodimer1, homodimer2 and homodimer3 form dimers only with themselves (e.g. homodimer1 pairs only with another homodimer1), but not with other types of homodimer (e.g. homodimer1 does not pair with homodimer2 or homodimer3).

Inventors discovered that combinations 1-4 are able to form a parallelepiped (cuboid), while combinations 5 and 6 can also form a tetrahedron but none of them can form an octahedron. Combination 1 can form prisms with an even length of sides, while combination 2 is not limited to the number of sides of prism of height 1 that they can form.

Inventors discovered that combination 7 can assemble into a tetrahedron, parallelepiped (cuboid) and can form a trigonal nano-network.

Inventor discovered that polypeptide chain comprising four coiled-coil segments can form nanostructures, such as for example nano-network, for example polypeptide comprising coiled-coil forming segments composed of: antiparallel heterodimer1A - antiparallel heterodimer2A - antiparallel heterodimer1B - antiparallel heterodimer 2B, where heterodimers1A and heterodimer1B form antiparallel coiled-coil segment and heterodimers2A and heterodimer2B form coiled coil segment.

Additionally also polypeptide chain comprising six coiled-coil forming segments can form nanostructures, such as for example nano-network, for example polypeptide comprising coiled-coil forming segments composed of six antiparallel heterodimers arranged in three pair with the following sequential arrangement: antiparallel heterodimer1A - antiparallel heterodimer2A - antiparallel heterodimer3A - antiparallel heterodimer1B - antiparallel heterodimer 2B, - antiparallel heterodimer 3B where heterodimers1A and heterodimer1B form antiparallel a coiled-coil segment and heterodimers2A and heterodimer2B form a coiled coil segment and heterodimers3A and heterodimer3B form a coiled coil segment.

Inventors discovered that the concentration of the polypeptide comprising coiled-coil elements can dictate the type of the nanostructure that can be formed. At higher protein concentrations, typically above 5 µg/ml, a protein network can be formed resulting in hexagonal or trigonal or tetragonal arrangement depending on the composition of coiled-coil forming segments within the polypeptide chain.

Additionally some cyclic permutations of coiled-coil forming elements within the polypeptide chain can also lead to the formation of nanostructures, such as for example a cyclic permutation of combination 1, where its cyclic permutation is combination 8, which can form a parallelepiped, prisms or hexagonal network.

Cyclic permutation denotes the sequences that are derived from the original sequence of coiled-coil forming segments by moving the last element to the first place and displacing each other segment one position to the right or by moving the first element to the last place and displacing each other segment one position to the left.

### Polypeptide assembly

The term nano-cage refers to the assembly of polypeptide chains that has the shape of cage with dimensions at the nanoscale, preferably between 2 and 200 nm and the inside of which is hollow. Examples of the cage include, but are not limited to the polygonal structures preferably forming a cuboid (parallelepiped), tetrahedron and prisms with coiled-coil segments as the edges.

The term nano-network refers to the assembly of polypeptide chains that is interconnected and can propagate in two or three dimensions. Examples include but are not limited to the trigonal, tetragonal, hexagonal lattice or network forming different polygons in the plane.

The term nano-tube refers to the assembly of polypeptide chains assembling nano-network which can propagate in one dimension, while in the other dimension the network is cyclically connected with a defined circumference . Examples include but are not limited to the hexagonal connectivity or network forming different polygons in the plane or in three dimensions.

The term polypeptide chain has a general meaning and refers to the sequence of aminoacids that are connected by peptid bond between each contributing aminoacids.

The term segment has a general meaning and refers to the aminoacid sequence of the polypeptide chain that forms a defined homogeneous structural and design unit.

### Coiled-coil segment and coiled-coil forming segment

The term "coiled-coil segment" has a general meaning and refers to a structural polypeptide motif comprising 2 or more helices that twist around each other forming an unperturbed super coil. Coiled-coil forming segment denotes a segment on a single polypeptide chain that has the potential to form a coiled-coil segment in combination with one or more specific coiled-coil forming segments. Coiled-coil forming segment contains a heptad repeat designated (a-b-c-d-e-f-g)ₙ approximately every two turns of a helix. "a" and "d" usually represent nonpolar, hydrophobic residues that are found at the interface of the two helices of the coiled-coil forming segments, "e" and "g" are solvent exposed polar residues that interact electrostatically, "b", "c" and "f" are hydrophilic and exposed to the solvent. Different amino acids at positions "a-g" of coiled-coil forming segments define oligomerization state, specify helix orientation and stability of coiled-coil segments. More specifically, the term coiled-coil segment in the description refers to naturally occurring or designed coiled-coil protein structure motifs which comprise typically between two and 50 heptads and can be parallel or antiparallel and can form homo- or hetero-oligomers.

The coiled coil forming segments could be selected from naturally occurring or designed coiled-coil protein structure motifs, preferentially from naturally occurring leucine-zipper proteins or from designed coiled-coil sequences, preferentially from SEQ ID: No. 2, 4, 6, 8, 10, 12, 14, 16, 18, 20, 22, 24, 26, 28.

The orientation of chains (coiled-coil-forming segments) in the coiled coil can be either parallel (all protein chains run in the same direction) or antiparallel (chains run in opposite directions). Principles determining the identification and properties of coiled-coil forming segments are well known to the researchers skilled in this subject and stability of parallel coiled-coil segments can be predicted from their sequence (Hagemann UB, Mason JM, Müller KM, Arndt KM., J.Mol.Biol. 381(1):73-88, 2008).

The term "homodimer" in the description of invention has a general meaning and refers to a complex composed of only one type of monomers.

The term "heterodimer" in the description of invention has a general meaning and refers to a complex composed of different type of monomers.

The main characteristics of coiled-coil forming segments selected for the inclusion into the same polypeptide chain is that each coiled-coil segment interacts only or with high preference with the segment and orientation it was designed for and not with any other segment in the reaction mixture. For example homodimeric coiled-coil should form coiled-coil only with another identical coiled-coil segment or heterodimeric parallel coiled coil segment should interact only with a corresponding pair segment, another defined heterodimeric coiled-coil segment.

Inventors discovered that polypeptide chains that form a nanonetwork covering surface can also form a nano-tube with its surface represented by a polypeptide network.

### Nanostructure assembly

Well designed nanostructures should represent the most stable of the possible assemblies, which is more stable than other types of the assemblies. For the assembly of a large number of components the energetic difference becomes relatively smaller and the system requires longer time to achieve the most stable arrangement. This can be accomplished by keeping the system under the conditions of a dynamic exchange between unfolded and assembled components. Those conditions are approximated by conditions where the approximately equal amount (approximately 50%) of the polypeptide chains if folded and the same amount is not assembled and is unfolded. Inventors discovered a procedure to improve the assembly of polypeptide nanostructures that allows sufficient time for the assembly of the most stable structures by a very slow decrease of the concentration of denaturing agent, where the polypeptide that forms nanostructures is dialyzed from the conditions where it is unfolded and disassembled against a slowly decreasing concentration of a dialyzing solution by slowly adding the buffer into the dialysis solution in the presence of rapid mixing. The refolding and nanostructure assembly thus occurs over a period of more than 12 hours, typically between 12 and 40 hours. Range of the starting and ending concentration of the dialyzing solution is determined from the spectroscopic analysis of the investigated polypeptide chain in the presence of different concentrations of denaturing agent. Different denaturing agents can be sued for this procedure, such as guanidinium hydrochloride, urea, LiBr or other chemical agents that disrupt the secondary structure of coiled-coil segments at high concentrations, typically 4-5 M guanidinium hydrochloride and nanostructure assembly occurs at lower concentrations, typically less than 1 M guanidninium hydrochloride.

In contrast to fusion proteins in U.S. Patent No. 6,756,039 where protein domains are rigidly linked by a rigid linker such as alpha helix, fusion proteins of the said invention contain flexibly linked protein domains that function like a molecular hinge allowing regulated degree of rotation of rigid coiled-coil segments.

The term "linker" refers to shorter amino acid sequences, whose role could be only to separate the individual domains or segments of the protein. Invention comprises the existence of nonhelical segments - linker - between coiled coil regions that break the helix and provide the flexibility for the assembly between different chains. Typically linker was composed of the Gly-Ser dipeptide. Linker segment typically span from 1 to 20 residues, preferentially between 1 and 8 amino-acid residues and preferentially it includes residues that break alpha-helix and are preferentially hydrophilic or are the site of additional function, such as binding metal ions, binding fluorescent tags or other molecules, such as drugs, preferential residues for the linker are glycine, serine, threonine, proline, cysteine, histidine, tryptophan, tyrosine.

Additional amino acid sequences at either end or between the coiled-coil forming segments, which are not necessary for formation of polypeptide material, could be appended to the polypeptide for example to simplify purification, allow detection or endow the material with additional functional properties.

The term "signal sequence" or "signal peptide" refers to the amino-acid sequence, which is important for directing the protein to a certain location in the cell. Signal sequences also vary depending on the host organism in which the fusion protein is expressed. Amino-acid sequences of the signal sequences are well known to experts, as well as which signal sequence is functional in a certain organism.

The term "tag sequence" refers to the sequence of amino acids, which is added to the protein to facilitate purification/isolation/detection of the protein.

The position of the signal sequence and tag sequence are optional but it must allow functional expression of the protein and maintain the function for which these amino acid sequences were selected, which is known to experts in the field.

The term "DNA" in the description refers to a sequence of nucleotides having an open reading frame that encodes a protein of the subject invention, being operatively linked to the regulatory elements, promoter and terminator to promote expression of the protein in the host cells. The length of the DNA sequence may vary greatly depending on the particular protein.

The term "regulatory elements" in the description has a general meaning and refers to the region of DNA in expression vector where regulatory proteins such as transcription factors bind and control gene expression.

The term "promoter" in the description has a general meaning and refers to a region of DNA in expression vector facilitating the transcription of target gene. The type of the promoter depends on host organism in which the gene will be expressed. In case of expression in prokaryotes, namely *E. coli,* several promoters such as P_{lac}, P_{T5} and preferentially P_{T7} can be used. In case of expression in eukaryotes, namely in mammalian cells, the promoters are in most cases of viral origin such as P_{CMV} or P_{SV40}.

The term "terminator" in the description has a general meaning and refers to the sequence that marks the end of a gene for transcription. The type of the terminator depends on host organism in which the gene will be expressed. In case of expression in prokaryotes, namely *E. coli,* a terminator from bacteriophage T7 can be used.

### Recombinant nucleic acid

Standard methods of molecular biology were used in the invention and are generally known to experts in the field (Sambrook et al. 1989. Molecular Cloning: A laboratory manual, 2nd ed., Cold Spring Harbor, NY, Ausubel et al. Current Protocols in Molecular Biology, Green Publishing Associates, Inc. and John Wiley & Sons, Inc., NY).

The invented proteins of the polypeptide material can be synthesized by expressing DNA coding for proteins, in a suitable host organism. The DNA coding for the proteins is inserted in an appropriate expression vector. Suitable vectors include, but are not limited to: plasmids, viral vectors, etc. Expression vectors, which are compatible with the host organism cells, are well known to experts in the field and include the appropriate control elements for transcription and translation of nucleic acid sequence. Typically, an expression vector includes an expression cassette, which includes in 5' to 3' direction the promoter, the coding sequence for the fusion protein operatively linked with the regulatory elements, promoter and terminator, including a stop codon for RNA polymerase and polyadenylation signal for polyadenylase.

Expression vector may be prepared for expression in prokaryotic and eukaryotic cells. For example, prokaryotic cells are bacteria, primarily *Escherichia coli.* As described herein, prokaryotic cells are used to get a sufficient quantity of nucleic acid. Expression vector generally contains the operationally associated control elements which are operationally linked to the DNA of the invention, which codes for the protein. The control elements are selected in a way to trigger efficient and tissue-specific expression. The promoter may be constitutive or inducible, depending on the desired pattern of expression. The promoter may be of native or foreign origin (not represented in the cells, where it is used), and may be natural or synthetic. The promoter must be chosen in order to work in the target cells of the host organism. In addition, initiation signals for the efficient translation of fusion protein are included, which comprises the ATG and the corresponding sequences. When the vector includes two or more reading frames, the reading frames should be operationally associated with control elements independently and the control elements should be the same or different, depending on the desired production of proteins.

Examples of bacterial expression vectors include, but are not limited to: pET vectors, pRSET vectors, and others. When vectors are used in the bacterial cells, the control elements are of bacterial origin.

Examples of mammalian expression vectors for mammalian cells include, but are not limited to: pcDNA (Invitrogen), pFLAG (Sigma), and others. When vectors are used in mammalian cells, the control elements are in most cases of viral origin, for example, adenovirus 2, cytomegalovirus, a virus Simian virus 40.

### Protein production/Process for preparation of polypeptide material

Invention also describes a process of generating the polypeptide material and includes introducing DNA coding protein of polypeptide material into the host organism, cultivating host organism under conditions suitable for protein expression, isolation of protein and exposing protein to specific conditions to form nanostructures.

A fusion protein can be synthesized in the host organism that expresses the heterologous nucleic acid, which encodes for the fusion protein. Invented fusion protein is used for preparation of polypeptide material. Optionally, a fusion protein is operatively linked to the signal sequence that is coded in the nucleic acid.

In general, the heterologous nucleic acid is incorporated into an expression vector (viral or non-viral), which is described above.

The invention describes host cells and organisms that contain nucleic acid according to the invention (transient or stable), which codes for the fusion protein according to the invention. Appropriate host organisms are known to the experts in the field of molecular biology and include bacterial and eukaryotic cells.

The transfer of vectors into host cells is carried out by conventional methods, known in the state of the art, and described above.

The DNA transfer may be transient or stable. Transient expression refers to the introduction of the vector DNA, which according to the invention is not incorporated into the genome of cells. Stable intake is achieved by incorporating DNA of the invention into the host genome. The transfer of the DNA according to the invention, especially for the preparation of the host organism, which has a stable DNA integrated, may according to the invention be controlled by the presence of markers. DNA coding for markers refers to resistance to drugs, for example antibiotics, and may be included in the vector with the DNA according to the invention, or on a separate vector.

Examples of implementation, designed to illustrate the invention, are shown below. The descriptions of examples of implementation have no intention of limiting the invention and should be understood as a demonstration of the invention. Further, these may be changed without deviating from the scope of the invention.

### Examples of implementation

### Example 1. Selection and design of orthogonal coiled-coil forming segments

The most important interactions governing the assembly of coiled-coil segments are well known to the experts in the field. Hydrophobic interactions at positions "a" and "d", electrostatic interactions at positions "e" and "g" and unfavorable burial of polar residues, such as Asn at positions "a" or "d" versus interaction with Asn at the same interacting position in the paired coiled-coil segment present the most important contributions defining the stability and selectivity of coiled coil pairs. Stability of coiled-coil segment can be estimated from the sequences based on the published algorithms (Hagemann UB, Mason JM, Müller KM, Arndt KM., J.Mol.Biol. 381(1):73-88, 2008). Formation of stable nanostructure according to this invention requires that the coiled-coil segments used in the design are orthogonal, meaning that two or more coiled-coil segments can form simultaneously in the solution with coiled-coil forming segments interacting only with its designed pairs and not with other coiled-coil forming segments. Pairs of coiled-coil forming segments were designed SEQ ID No: 12, 14, 16, 18, 20, 22, 24, 26, where the stability and specificity of pairs were designed by combinations of distribution of differently charged resides along with positioning of Asn residue pairs at positions that favors formation of pairs and destabilizes unwanted pairs. Tyrosine residues is added to facilitate detection and quantification of the peptide by UV absorbance. SPED tetrapeptide is added to the N-terminus since those residues favor initiation of helix at the N-terminus.

**Table 1: Sequences of designed coiled-coil forming segments designed in orthogonal pairs P1-P2, P3-P4, P5-P6 and P7-P8. Asn residues are inserted twice in each segment at the equivalent "a" positions of the same pair of coiled-coil forming segments, differing from the positions of other coiled-coil forming segments and contributing to the pairing specificity are underlined. Charged residues governing electrostatic interactions between pairs of coiled-coil forming segments are shown in bold.**

| | |
|---|---|
| P1 | SPED**E**IQAL**E**E**EN**AQL**E**Q**EN**AAL**E**E**E**IAQL**E**YG |
| P2 | SPED**K**IAQL**K**E**KN**AAL**K**E**KN**QQL**K**E**K**IQAL**K**YG |
| P3 | SPED**E**IQQL**E**E**E**IAQLEQ**KN**AALRE**RN**QAL**K**YG |
| P4 | SPED**K**IAQL**K**Q**K**IQAL**K**Q**EN**QQL**E**E**EN**AAL**E**YG |
| P5 | SPED**EN**AAL**E**E**K**IAQL**K**Q**KN**AAL**K**E**E**IQAL**E**YG |
| P6 | SPED**KN**AAL**K**E**E**IQAL**E**E**EN**QAL**E**E**K**IAQL**K**YG |
| P7 | SPED**E**IQAL**E**E**KN**AQL**K**Q**E**IAAL**E**E**KN**QAL**K**YG |
| P8 | SPED**K**IAQL**K**E**EN**QQL**E**Q**K**IQAL**K**E**EN**AAL**E**YG |

### Example 2. Design and preparation of DNA constructs

DNA sequences for polypeptide domains described above were designed from amino-acid sequences of the selected polypeptide domains using tool Designer from DNA2.0 Inc. that enables the user to design DNA fragments and optimize expression for the desired hosts (e.g. *E .coli*) using codon optimization. Genes were then ordered from MR.Gene GmbH (Im Gewerbepark B32, D-93059 Regensburg), cut out with restriction endonucleases (restriction enzymes) and cloned into the appropriate vector containing necessary regulatory sequences known to the experts in the field. Vectors used include commercial vectors pET, pRSETA and pSB1A2 (pSB1A2 http://partsregistry.org/Part:pSB1A2), carrying all necessary features such as antibiotic resistance, origin of replication and multiple cloning site.

Molecular biology methods (DNA fragmentation with restriction endonucleases, DNA amplification using polymerase chain reaction-PCR, PCR ligation, DNA concentration detection, agarose gel electrophoresis, purification of DNA fragments from agarose gels, ligation of DNA fragments into a vector, transformation of chemically competent cells *E.coli* DH5α, isolation of plasmid DNA with commercially available kits, screening and selection) were used for preparation of DNA constructs. All procedures were preformed under sterile conditions (aseptic technique). DNA segments were characterized by restriction analysis and sequencing.

Molecular cloning procedures are well known to the experts in the field and are described in details in molecular biology handbook (Sambrook J., Fritsch E.F., Maniatis T. 1989. Molecular cloning: A laboratory manual. 2nd ed. New York, Cold Spring Harbor Laboratory Press: 1659 p.).

### Example 3: Preparation of recombinant polypeptide consisting of three coiled-coil segments

DNA constructs coding for the polypeptide comprising three coiled-coil forming segments were prepared to demonstrate the feasibility of production of fusion proteins composed of a three coiled-coil forming segments. We selected the polypeptide chain that contains a parallel heterodimerization domain A, parallel homodimerization domain and a parallel heterodimerization domain B, where heterodimer A and heterodimer B form a coiled-coil heterodimer. Coiled-coil forming segments were selected among the SEQ ID: No 12, SEQ ID: no 8 and SEQ ID: No 14 with Gly-Ser dipeptide linker between those domains. Additionally a hexahistidine peptide tag was added to the polypeptide chain in order to facilitate isolation, detection and binding of metal ions.

Additionally the peptide linker between coiled-coil forming segments could be varied from 1 to 40 amino acids and His₆-tag could be positioned either on N- terminus or C-terminus or could be replaced with a similar peptide tag.

Plasmids encoding open reading frames of fusion proteins from Table 3 were transformed into chemically competent *E. coli* BL21 (DE3) pLysS cells. Selected bacterial colonies grown on LB plates supplemented with selected antibiotic (ampicillin, kanamycin) were inoculated into 100 mL of LB growth media supplemented with antibiotic and grown overnight at 37°C. Next day the overnight culture was diluted 20-50-times reaching OD₆₀₀ of diluted culture between 0.1 and 0.2. The bacterial culture was grown until OD₆₀₀ reached 0.6 - 0.8 when protein expression was induced by the addition of inducer IPTG (from 0.4 mM to 1 mM). Two hours after induction culture broth was centrifuged and bacterial cells were resuspended in lysis buffer (Tris pH 8.0, 0.1 % deoxycholate supplemented with protease inhibitor cocktail) and frozen at -80°C. Thawed cell suspension was further lysed by sonication and centrifuged. Precipitate (residual non-lysed cells, inclusion bodies) and supernatant were checked for expression of constructs by SDS-PAGE and when necessary by Western blot using anti-His-tag antibodies as primary antibodies. Produced recombinant polypeptide was mainly present in insoluble part (inclusion bodies), which was composed of >80% of the overexpressed protein. Inclusion bodies were washed several times with lysis buffer, twice with 2 M urea in 10 mM Tris pH 8.0 and once with MiliQ water. Usually this treatment resulted in >90% of protein purity (Figure 6). In cases where the precipitated material still contained impurities, inclusion bodies were dissolved in 6 M GdnHCl pH 8.0 and loaded on Ni²⁺-NTA columns (Quiagen, GE). Purification under denaturing conditions was followed according to the manufacturer's instructions. After elution with 250 mM imidazole pH 5.8 fractions containing protein were combined and dialyzed twice against 10 mM Hepes pH 7.5 or other appropriate buffer. Fractions containing protein were combined and analyzed by SDS PAGE and western blot.

### Example 4: Determination of the unfolding characteristics of polypeptide SEQ ID: No 28

Polypeptide SEQ ID: No 28 dissolved in 6 M GdnHCl was diluted to the final concentration of 0.1 mg/ml in 20 mM HEPES buffer solutions pH 7.0, containing different concentrations of guanidinium hydrochloride, from 6 M to the solution without denaturing agent. Secondary structure of the polypeptide at different concentrations was determined by measuring the circular dichroism spectra between 320 and 190 nm on a CD spectrometer (Figure 7).

The fraction of folded and unfolded protein was determined from the ratio of molar ellipticity at each concentration of denaturing agent and the maximal ellipticity in the solution without denaturing agent (Figure 8)

### Example 5: Refolding of polypeptide SEQ ID: No 28 at low concentration

Purified polypeptide SEQ ID: no 28 in 6 M guanidinium hydrochloride was diluted to 0.5 µg/ml in 5 M guanidinium hydrochloride in 20 mM HEPES buffer pH 7.5. 50 ml of the solution was placed in a dialysis bag with membrane cutoff of 3.5 kDa, and placed in 2 1 beaker containing a 200 ml of the 5 M guanidinium hydrochloride in 20 mM HEPES buffer pH 7.5. Solution was stirred at room temperature with magnetic mixer at 1000 rpm. To the bottom of the dialysis solution in the vicinity of rotating magnet a solution with 20 mM HEPES pH 7 was added by a pump set to the flow rate of 1.4 ml/min. Within 12 hours the concentration of denaturing agent in the solution decreased to 1 M. At this point the dialysis bag with protein solution was transferred to the solution containing 21 or 20 mM HEPES pH 7.5 and dialyzed for 3 hours and the procedure was repeated twice, to decrease the content of denaturant.

Sample with the resulting self-assembled nanostructure was analyzed by atomic force microscope, which demonstrated the presence of small nanoparticles, measuring less than 10 nm, which was according to the predictions of the self-assembled cuboid (parallelepiped) with edges composed of P1-P2 and GCN-homodimeric coiled-coil segments (Figure 9). Nanogold reagent (Nanoprobes, Yaphank, NY, USA), and silver enhancement reagent (Nanoprobes, Yaphank, NY, USA) that binds to the hexahistidine tag increased the size of particles were added to the sample, according to the instructions from the producer, demonstrating the accessibility of the hexahistidine tags in the self-assembled nanostructure.

### Example 6: Refolding of polypeptide SEQ ID: No 28 at higher concentration

Isolated polypeptide SEQ ID: no 28 in 6 M guanidinium hydrochloride was diluted to 50 µg/ml in 5 M guanidinium hydrochloride in 20 mM HEPES buffer pH 7.5. 50 ml of the solution was placed in a dialysis bag with membrane cutoff of 5 kDa and placed in 2 1 beaker containing a 200 ml of the 5 M guanidinium hydrochloride in 20 mM HEPES buffer pH 7.5. Solution was mixed at room temperature with magnetic mixer at 1000 rpm. To the bottom of the dialysis solution in the vicinity of rotating magnet a solution with 20 mM HEPES pH 7 was added by a pump set to the flow rate of 1.4 ml/min. In 12 hours the concentration of denaturing agent in the solution decreased to 1 M. At this point the dialysis bag with protein solution was transferred to the solution containing 21 and 20 mM HEPES pH 7.5 and dialyzed for 3 hours and the procedure was repeated twice, to decrease the content of denaturant.

Sample with the resulting self-assembled nanostructure was analyzed by transmission electon microscope. Sample of the self assembled nanostructure made of SEQ ID: No 28 was deposited on the grid for TEM analysis and stained with uranyl acetate. Image from the transmission electron microscope demonstrated formation of molecular network with edges at nanometer dimensions consistent with multiples of the designed edges consisting of P-1P2 and GCNp1-GCNp1 coiled-coils (Figure 10).

### Industrial applicability

Nano-cages made of polypeptides could be used to encase nanoparticles that can be used for different applications, such as drug delivery, chemical catalysis within the nanocage, particularly since the side chains of the polypeptide chain that do not invalidate coiled-coil interactions can be selected to perform specific interactions at selected positions.

Self-assembled polypeptide network can be used to bind specific compounds, either within pores of the assembled network or at the vertices, that contain linkers and N- or C-terminal segments of the building polypeptides, which can incorporate various linkers, such as hexahistidine or other peptide tags. Polypeptide network can be used as a scaffold for various functions, such as for example, but not limited to binding carbon nanotubes, biomineralization, metal binding, formation of conducting circuits, formation of optical assemblies at the nanoscale, combinations with other molecular assemblies, binding of antibodies, binding of nucleic acids, binding of hydrophobic groups for anchoring to the membrane, ultrafiltration, ...

## Claims

1. Polypeptide comprising any of the following arrangements of coiled-coil forming segments:
a. parallel heterodimer-forming segment A - parallel homodimer-forming segment - parallel heterodimer-forming segment B,
b. parallel heterodimer-forming segment A - antiparallel homodimer-forming segment - parallel heterodimer-forming segment B and its cyclic permutations,
c. antiparallel heterodimer-forming segment A - parallel homodimer-forming segment - antiparallel heterodimer-forming segment B,
d. antiparallel heterodimer-forming segment A - antiparallel homodimer-forming segment - antiparallel heterodimer-forming segment B,
e. parallel homodimer-forming segment 1 - antiparallel homodimer-forming segment - parallel homodimer-forming segment 2,
f. antiparallel homodimer-forming segment 1 - parallel homodimer-forming segment - antiparallel homodimer-forming segment 2 and its cyclic permutations,
g. antiparallel homodimer-forming segment 1 - antiparallel homodimer-forming segment 2 - antiparallel homodimer-forming segment 3,
h. parallel homodimer-forming segment 1 - parallel homodimer-forming segment 2 - parallel homodimer-forming segment 3 - parallel heterodimer-forming segment A - parallel heterodimer-forming segment B - antiparallel homodimer-forming segment
i. antiparallel heterodimer-forming segment 1 A - antiparallel heterodimer-forming segment 2A - antiparallel heterodimer-forming segment 1B - antiparallel heterodimer-forming segment 2B, or
j. antiparallel heterodimer-forming segment 1A- antiparallel heterodimer-forming segment 2A - antiparallel heterodimer-forming segment 3A - antiparallel heterodimer-forming segment 1B - antiparallel heterodimer-forming segment 2B - antiparallel heterodimer-forming segment 3B,
wherein a pair of heterodimer-forming segment A and heterodimer-forming segment B form a heterodimeric coiled-coil; homodimer-forming segments form coiled-coils with an identical homodimer-forming segment and homodimer-forming segment 1, homodimer-forming segment 2 and homodimer-forming segment 3 form dimers only with themselves (e.g. homodimer-forming segment 1 pairs with another homodimer-forming segment 1), but not with other types of homodimer (e.g. homodimer-forming segment 1 does not pair with homodimer-forming segment 2); and wherein heterodimer-forming segment 1A and heterodimer-forming segment 1 B form antiparallel coiled-coils; and heterodimer-forming segment 2A and heterodimer-forming segment 2B form coiled coils; and heterodimer-forming segment 3A and heterodimer-forming segment 3B form coiled coils;
further wherein each coiled-coil forming segment comprises two to fifty heptads; and further wherein a non-helical peptide linker is added between the coiled-coil forming segments, the length of said linker being between 1 and 40 amino acid residues.

2. Polypeptide according to claim 1, wherein the coiled-coil forming segment is selected from the coiled-coil forming segment of natural protein or designed protein, and is preferentially selected from SEQ ID No.: 2, 4, 6, 8, 10, 12, 14, 16, 18, 20, 22, 24, and 26.

3. Polypeptide according to claim 1 or 2, wherein the length of the linker is between 1 and 8 amino-acid residues.

4. Polypeptide according to any of claims 1 to 3, prepared by a recombinant method.

5. Nanostructure, comprising the polypeptide of any of claims 1-4.

6. Nanostructure according to claim 5, wherein the structure is nano-cage, nano-network or nano-tube.

7. Polynucleotide coding for a coiled-coil forming segment, wherein the polynucleotide has a nucleotide sequence selected from SEQ ID Nos. 1, 3, 5, 7, 9, 11, 13, 15, 17, 19, 21, 23, and 25.

8. DNA coding for a polypeptide according to any of claims 1 to 4, wherein the DNA is operatively linked to the regulatory elements, promoter and terminator, which drive expression of fusion proteins in the host cells.

9. Procedure for the assembly of a polypeptide nanostructure according to claim 5 or 6, comprising the step of dialysing the polypeptide against a dialysis solution for at least 12 hours using slowly decreasing concentrations of a denaturing agent, or against a dialysis solution in which the concentration of the denaturing agent is stepwise decreased in more than five, typically between five and ten, steps.

10. Use of the polypeptide of any of claims 1 to 3 for forming the nanostructure of claim 5 or 6.

11. Use of the polypeptide of any of claims 1 to 3 or the nanostructure of claim 5 or 6 as a molecular carrier to encase nanoparticles.

12. The nanostructure according to claim 6, wherein the nanostructure is a nano-cage, for use in drug delivery or in a chemical catalysis within the nano-cage.

13. The nanostructure according to claim 6, wherein the nanostructure is a nano-network, for use in binding specific compounds or as a scaffold.

14. The nanostructure for the use according to claim 13, wherein the nano-network is for binding carbon nanotubes, biomineralization, metal binding, formation of conducting circuits, formation of optical assemblies at the nanoscale, combinations with other molecular assemblies, binding of antibodies, binding of nucleic acids, binding of hydrophobic groups for anchoring to the membrane, or ultrafiltration.

## Patentansprüche

1. Polypeptid, umfassend eine der folgenden Gruppierungen Coiled-Coil-bildender Abschnitte:
a. ein paralleles Heterodimer bildender Abschnitt A - ein paralleles Homodimer bildender Abschnitt - ein paralleles Heterodimer bildender Abschnitt B,
b. ein paralleles Heterodimer bildender Abschnitt A - ein antiparalleles Homodimer bildender Abschnitt - ein paralleles Heterodimer bildender Abschnitt B und dessen zyklische Permutationen,
c. ein antiparalleles Heterodimer bildender Abschnitt A - ein paralleles Homodimer bildender Abschnitt - ein antiparalleles Heterodimer bildender Abschnitt B,
d. ein antiparalleles Heterodimer bildender Abschnitt A - ein antiparalleles Homodimer bildender Abschnitt - ein antiparalleles Heterodimer bildender Abschnitt B,
e. ein paralleles Homodimer bildender Abschnitt 1 - ein antiparalleles Homodimer bildender Abschnitt - ein paralleles Homodimer bildender Abschnitt 2,
f. ein antiparalleles Homodimer bildender Abschnitt 1 - ein paralleles Homodimer bildender Abschnitt - ein antiparalleles Homodimer bildender Abschnitt 2 und dessen zyklische Permutationen,
g. ein antiparalleles Homodimer bildender Abschnitt 1 - ein antiparalleles Homodimer bildender Abschnitt 2 - ein antiparalleles Homodimer bildender Abschnitt 3,
h. ein paralleles Homodimer bildender Abschnitt 1 - ein paralleles Homodimer bildender Abschnitt 2 - ein paralleles Homodimer bildender Abschnitt 3 - ein paralleles Heterodimer bildender Abschnitt A - ein paralleles Heterodimer bildender Abschnitt B - ein antiparalleles Homodimer bildender Abschnitt,
i. ein antiparalleles Heterodimer bildender Abschnitt 1A - ein antiparalleles Heterodimer bildender Abschnitt 2A - ein antiparalleles Heterodimer bildender Abschnitt 1B - ein antiparalleles Heterodimer bildender Abschnitt 2B oder
j. ein antiparalleles Heterodimer bildender Abschnitt 1A - ein antiparalleles Heterodimer bildender Abschnitt 2A - ein antiparalleles Heterodimer bildender Abschnitt 3A - ein antiparalleles Heterodimer bildender Abschnitt 1B - ein antiparalleles Heterodimer bildender Abschnitt 2B - ein antiparalleles Heterodimer bildender Abschnitt 3B,
wobei ein Paar aus einem Heterodimer-bildenden Abschnitt A und einem Heterodimer-bildenden Abschnitt B ein heterodimeres Coiled-Coil bilden; Homodimerbildende Abschnitte Coiled-Coils mit einem identischen Homodimer-bildenden Abschnitt bilden und ein Homodimer-bildender Abschnitt 1, ein Homodimer-bildender Abschnitt 2 und ein Homodimer-bildender Abschnitt 3 Dimere nur mit sich selbst (z. B. paart ein Homodimer-bildender Abschnitt 1 mit einem weiteren Homodimer-bildenden Abschnitt 1), aber nicht mit anderen Arten von Homodimeren bilden (z. B. paart ein Homodimer-bildender Abschnitt 1 nicht mit einem Homodimer-bildenden Abschnitt 2);
und wobei der Heterodimer-bildende Abschnitt 1A und der Heterodimer-bildende Abschnitt 1 B antiparallele Coiled-Coils bilden; und der Heterodimer-bildende Abschnitt 2A und der Heterodimer-bildende Abschnitt 2B Coiled-Coils bilden; und der Heterodimer-bildende Abschnitt 3A und der Heterodimer-bildende Abschnitt 3B Coiled-Coils bilden; wobei ferner jeder Coiled-Coil-bildende Abschnitt zwei bis fünfzig Heptaden umfasst; und wobei ferner ein nicht helikaler Peptidlinker zwischen den Coiled-Coil-bildenden Abschnitten hinzugefügt ist, wobei die Länge des Linkers zwischen 1 und 40 Aminosäureresten beträgt.

2. Polypeptid gemäß Anspruch 1, wobei der Coiled-Coil-bildende Abschnitt aus dem Coiled-Coil-bildenden Abschnitt eines natürlichen Proteins oder künstlichen Proteins ausgewählt ist und vorzugsweise aus SEQ ID NO: 2, 4, 6, 8, 10, 12, 14, 16, 18, 20, 22, 24 und 26 ausgewählt ist.

3. Polypeptid gemäß Anspruch 1 oder 2, wobei die Länge des Linkers zwischen 1 und 8 Aminosäurereste beträgt.

4. Polypeptid gemäß einem der Ansprüche 1 bis 3, hergestellt durch ein rekombinantes Verfahren.

5. Nanostruktur, umfassend das Polypeptid nach einem der Ansprüche 1 - 4.

6. Nanostruktur gemäß Anspruch 5, wobei es sich bei der Struktur um einen Nanokäfig, ein Nanonetzwerk oder eine Nanoröhre handelt.

7. Polynucleotid, welches einen Coiled-Coil-bildenden Abschnitt kodiert, wobei das Polynucleotid eine Nucleotidsequenz aufweist, ausgewählt aus SEQ ID NO: 1, 3, 5, 7, 9, 11, 13, 15, 17, 19, 21, 23 und 25.

8. DNA, welche ein Polypeptid gemäß einem der Ansprüche 1 bis 4 kodiert, wobei die DNA mit den regulatorischen Elementen, Promotor und Terminator, funktionell verknüpft ist, welche die Expression von Fusionsproteinen in den Wirtszellen antreiben.

9. Verfahren zum Anordnen einer Polypeptid-Nanostruktur gemäß Anspruch 5 oder 6, umfassend den Schritt, das Polypeptid gegen eine Dialyselösung mindestens 12 Stunden lang unter Verwendung langsam abnehmender Konzentrationen eines denaturierenden Mittels oder gegen eine Dialyselösung, in der die Konzentration des denaturierenden Mittels schrittweise in mehr als fünf, normalerweise zwischen fünf und zehn Schritten gesenkt wird, zu dialysieren.

10. Verwendung des Polypeptids nach einem der Ansprüche 1 bis 3 zum Bilden der Nanostruktur nach Anspruch 5 oder 6.

11. Verwendung des Polypeptids nach einem der Ansprüche 1 bis 3 oder der Nanostruktur nach Anspruch 5 oder 6 als molekularen Träger zum Einkapseln von Nanopartikeln.

12. Nanostruktur gemäß Anspruch 6, wobei es sich bei der Nanostruktur um einen Nanokäfig handelt, zur Verwendung bei der Arzneimittelabgabe oder bei einer chemischen Katalyse innerhalb des Nanokäfigs.

13. Nanostruktur gemäß Anspruch 6, wobei es sich bei der Nanostruktur um ein Nanonetzwerk handelt, zur Verwendung beim Binden spezifischer Verbindungen oder als Gerüst.

14. Nanostruktur zur Verwendung gemäß Anspruch 13, wobei das Nanonetzwerk zum Binden von Kohlenstoff-Nanoröhren, zur Biomineralisierung, Metallbindung, Bildung leitender Stromkreise, Bildung optischer Anordnungen im Nanomaßstab, für Kombinationen mit anderen molekularen Anordnungen, zum Binden von Antikörpern, Binden von Nucleinsäuren, Binden hydrophober Gruppen zum Verankern mit der Membran oder zur Ultrafiltration bestimmt ist.

## Revendications

1. Polypeptide comprenant l'un quelconque des agencements suivants de segments formant une superhélice (coiled-coil):
a. segment formant un hétérodimère parallèle A - segment formant un homodimère parallèle - segment formant un hétérodimère parallèle B,
b. segment formant un hétérodimère parallèle A - segment formant un homodimère antiparallèle - segment formant un hétérodimère parallèle B et ses permutations cycliques,
c. segment formant un hétérodimère antiparallèle A - segment formant un homodimère parallèle - segment formant un hétérodimère antiparallèle B,
d. segment formant un hétérodimère antiparallèle A - segment formant un homodimère antiparallèle - segment formant un hétérodimère antiparallèle B,
e. segment formant un homodimère parallèle 1 - segment formant un homodimère antiparallèle - segment formant un homodimère parallèle 2,
f. segment formant un homodimère antiparallèle 1 - segment formant un homodimère parallèle - segment formant un homodimère antiparallèle 2 et ses permutations cycliques,
g. segment formant un homodimère antiparallèle 1 - segment formant un homodimère antiparallèle 2 - segment formant un homodimère antiparallèle 3,
h. segment formant un homodimère parallèle 1 - segment formant un homodimère parallèle 2 - segment formant un homodimère parallèle 3 - segment formant un hétérodimère parallèle A - segment formant un hétérodimère parallèle B - segment formant un homodimère antiparallèle,
i. segment formant un hétérodimère antiparallèle 1A - segment formant un hétérodimère antiparallèle 2A - segment formant un hétérodimère antiparallèle 1B - segment formant un hétérodimère antiparallèle 2B, ou
j. segment formant un hétérodimère antiparallèle 1A - segment formant un hétérodimère antiparallèle 2A - segment formant un hétérodimère antiparallèle 3A - segment formant un hétérodimère antiparallèle 1B - segment formant un hétérodimère antiparallèle 2B - segment formant un hétérodimère antiparallèle 3B,
dans lequel une paire constituée par un segment formant un hétérodimère A et un segment formant un hétérodimère B forment une superhélice hétérodimérique ; des segments formant un homodimère forment des superhélices avec un même segment formant un homodimère ; et le segment formant un homodimère 1, le segment formant un homodimère 2 et le segment formant un homodimère 3 forment uniquement des dimères avec eux-mêmes (par exemple le segment formant un homodimère 1 s'apparie avec un autre segment formant un homodimère 1), mais pas avec d'autres types d'homodimères (par exemple le segment formant un homodimère 1 ne s'apparie pas avec le segment formant un homodimère 2) ; et dans lequel le segment formant un hétérodimère 1A et le segment formant un hétérodimère 1B forment des superhélices antiparallèles ; et le segment formant un hétérodimère 2A et le segment formant un hétérodimère 2B forment des superhélices ; et le segment formant un hétérodimère 3A et le segment formant un hétérodimère 3B forment des superhélices ;
dans lequel en outre chaque segment formant une superhélice comprend de deux à cinquante heptades ; et dans lequel en outre un segment de liaison peptidique non hélicoïdal est ajouté entre les segments formant une superhélice, la longueur dudit segment de liaison étant comprise entre 1 et 40 résidus acide aminé.

2. Polypeptide selon la revendication 1, dans lequel le segment formant une superhélice est sélectionné à partir du segment formant une superhélice d'une protéine naturelle ou d'une protéine artificielle, et est de préférence sélectionné parmi les segments SEQ ID N° 2, 4, 6, 8, 10, 12, 14, 16, 18, 20, 22, 24 et 26.

3. Polypeptide selon la revendication 1 ou 2, dans lequel la longueur du segment de liaison est comprise entre 1 et 8 résidus acide aminé.

4. Polypeptide selon l'une quelconque des revendications 1 à 3, préparé par un procédé recombinant.

5. Nanostructure comprenant le polypeptide selon l'une quelconque des revendications 1 à 4.

6. Nanostructure selon la revendication 5, dans laquelle la structure est une nanocage, un nanoréseau ou un nanotube.

7. Polynucléotide codant pour un segment formant une superhélice, dans lequel le polynucléotide comporte une séquence de nucléotides sélectionnée parmi les séquences SEQ ID N° 1, 3, 5, 7, 9, 11, 13, 15, 17, 19, 21, 23 et 25.

8. ADN codant pour un polypeptide selon l'une quelconque des revendications 1 à 4, dans lequel l'ADN est lié de façon opérationnelle aux éléments régulateurs, promoteur et terminateur, qui commandent l'expression de protéines de fusion dans les cellules hôtes.

9. Procédure pour l'assemblage d'une nanostructure polypeptidique selon la revendication 5 ou 6, comprenant l'étape de dialyse du polypeptide contre une solution de dialyse durant au moins 12 heures en utilisant des concentrations lentement réduites d'un agent dénaturant, ou contre une solution de dialyse dans laquelle la concentration de l'agent dénaturant est réduite graduellement en plus de cinq étapes, soit typiquement entre cinq et dix étapes.

10. Utilisation du polypeptide selon l'une quelconque des revendications 1 à 3 pour former la nanostructure selon la revendication 5 ou 6.

11. Utilisation du polypeptide selon l'une quelconque des revendications 1 à 3 ou de la nanostructure selon la revendication 5 ou 6 comme porteur moléculaire pour envelopper des nanoparticules.

12. Nanostructure selon la revendication 6, dans laquelle la nanostructure est une nanocage destinée à être utilisée pour l'administration d'un médicament ou dans une catalyse chimique à l'intérieur de la nanocage.

13. Nanostructure selon la revendication 6, dans laquelle la nanostructure est un nanoréseau destiné à être utilisé pour lier des composés spécifiques ou comme squelette.

14. Nanostructure destinée à une utilisation selon la revendication 13, dans laquelle le nanoréseau est destiné à être utilisé pour la liaison de nanotubes de carbone, une biominéralisation, une liaison métallique, la formation de circuits conducteurs, la formation d'assemblages optiques nanométriques, des combinaisons avec d'autres assemblages moléculaires, la liaison d'anticorps, la liaison d'acides nucléiques, la liaison de groupes hydrophobes pour un ancrage à la membrane, ou une ultrafiltration.
